# EUROPEAN PATENT APPLICATION

(11) **EP 2 949 319 A1**
(43) Date of publication of application: **02.12.2015**
(21) Application number: 15169175.5
(22) Date of filing: 26.05.2015
(51) Int. Cl.: A61K 9/16, A61K 9/48, A61K 9/50

(54) **PHARMACEUTICAL COMPOSITIONS COMPRISING AN ACTIVE AGENT**

(30) Priority: 26.05.2014 EP 14169911
(71) Applicant: Galenicum Health S.L., 08005 Barcelona (ES)
(72) Inventor: ARROYO HIDALGO, Sergio, 08005 Barcelona (ES)
(74) Representative: Galenicum Health S.L.

(57) **Abstract**

The present invention relates to hard shell capsules comprising tamsulosin in the form of granules and an immediate release soft gelatin capsule comprising 7-beta-N-[2,5-bis(trifluoromethyl)]phenylcarbamoyl-4-aza-5-alpha-androst-1-en-3-one The invention further relates to processes for preparing such hard shell capsules.

## Description

The present invention relates to immediate release soft gelatin capsules comprising 17-beta-N-[2,5-bis(trifluoromethyl)]phenylcarbamoyl-4-aza-5-alpha-androst-1-en-3-one. The invention further relates to processes for preparing such soft gelatin capsules.

### STATE OF THE ART

Some pharmacologically active substances may have biopharmaceutical and/or physicochemical properties which make them difficult to formulate into commercially acceptable formulations. Such substances may however be conveniently administered in liquid form, e.g. in a complex carrier medium made up of several components. Liquid ingredients are difficult to include in any other solid dosage form such as a tablet.

Pharmaceutical dosage forms including liquids can be delivered in a variety of forms, for example but not exclusively, in a capsule. Gelatin capsules are solid dosage forms in which therapeutic agents are enclosed in a soluble gelatin wall. The wall can be made of either soft or hard gelatin.

Liquid filled hard gelatin capsules have also been used. See, for example, D. Cade et. al., Liquid Filled and Sealed Hard Gelatin Capsules, Drug Development and Industrial Pharmacy, 12(11-13): 2289-2300, (1986).

Soft gelatin capsules ("SGC" or soft gels) comprise a soft, globular, gelatin wall. Soft gelatin capsules offer a convenient dosage form for the administration of drugs, nutrients, vitamins, foodstuff and cosmetics. Soft gelatin capsules are one-piece and hermetically sealed to enclose normally a liquid or semi-liquid fill. Upon ingestion by the consumer (or on contact with water), moisture causes the capsule to come apart at the seams where the two halves are joined thereby releasing the fill or contents of the capsule.

Encapsulation of liquid formulations in soft gelatin capsules potentially offers a very convenient way of administering such pharmacologically active substances. However, the manufacture of commercially acceptable liquid filled soft gelatin capsules is fraught with difficulties which restrict the availability of this approach. The carrier medium may be designed to form an emulsion/solution in the stomach thereby facilitating absorption of the pharmacologically active substance. The carrier medium may have to be accurately prepared in order not to destroy the beneficial properties of the pharmacologically active substance or of the soft gelatin capsule. For example, the solubilizing properties of the capsule fill may be changed in such a way that the active substance may precipitate out. This precipitation process may be irreversible, and, as a consequence, the patient may be under-dosed. Also, the properties of the capsule fill may be changed, and, upon administration, the pharmacologically active substance may not be correctly or reproducibly absorbed. Moreover, during manufacture, the capsule shell is formed from wet gelatin bands and the resultant wet capsules are dried. During this stage or even afterwards, components in the capsule fill may migrate into the capsule shell, and vice versa, thereby changing the composition of the capsule fill at least in the boundary region near the interface of the capsule fill and the capsule shell, with the result that the beneficial properties of the capsule fill are lost. Furthermore, such softened capsules will undergo deformation, because due to the migration of part of the solvent into the capsule shell from the capsule fill there will be a decrease in volume and a reduction in pressure in the interior of the capsule. These solubility challenges can affect bioavailability possibly resulting in reduced or unpredictable bioavailability.

The lack of stability of a soft gelatin capsule can affect the dissolution profile of such capsule. In most of the cases, it is important that the active ingredient is delivered as soon as possible in order to have a rapid onset of action. These kinds of compositions are known as immediate release dosage forms. An immediate release dosage form should have the following characteristics to be marketed: It should dissolve and/or disintegrate in the stomach within a short period; It should be compatible with taste masking; It should be portable without fragility concern; It should have a pleasing mouth feel; It should leave minimal or no residue in the mouth after oral administration; It should exhibit low sensitivity to environmental condition as humidity and temperature; It should be manufactured using conventional processing and packaging equipment at low cost; It should show rapid dissolution and absorption profiles of the active ingredient, which may produce rapid onset of action.

The choice of excipients is important in order to ensure good solubility and good bioavailability of the pharmaceutically active compound when using soft gelatin capsules. See For example, K. Hutchison, Encapsulation in Softgels for Pharmaceutical Advantage, Spec. Pub. R. Soc. Chem., Vol. 138, pp. 86-97, (1993).

Methods for preparation of soft gelatin capsules are known. See, for example, J.P. Stanley, Soft Gelatin Capsules, Ch. 13 - Part Two in: The Theory and Practice of Industrial Pharmacy, eds. L. Lachman et. al., 3rd Ed., pp. 398-412, 1986, and W.R. Ebert, Soft Elastic Gelatin Capsules: A Unique Dosage Form, Pharmaceutical Technology, Vol. 1, No. 5. Soft gelatin capsules can be prepared, among other methods, by two methods. In short, the first method is known as the plate process, where a set of molds is used. A warm sheet of gelatin is laid over a lower plate and the liquid fill is poured on it. A second sheet of gelatin is then placed on top followed by the top plate. The set is placed under pressure to form the desired capsule. In sum, the second method was invented by Robert P. Scherer in 1933 and is called the rotary-die process. In this process, soft gelatin capsules are made by continuously casting two separate ribbons of molten or flowable gelatin into two separate rotating dies of an encapsulation machine to produce soft, elastic gelatin capsules.

Aza steroids are an important class of pharmaceutically active compounds. In particular there are 4-aza steroids and 6-aza steroids known to be inhibitors of the enzyme testosterone 5-alpha-reductase. Such compounds are useful in the treatment of benign prostatic hyperplasia, prostate cancer and other diseases. Dutasteride, a synthetic 4-aza steroid compound is an antiandrogen with the chemical name 17-beta-N-[2,5-bis(trifluoromethyl)]phenylcarbamoyl-4-aza-5-alpha-androst-1-en-3-one which is the steroid that is disclosed in U.S. Pat. N°. 5,565,467 (Batchelor et al.)

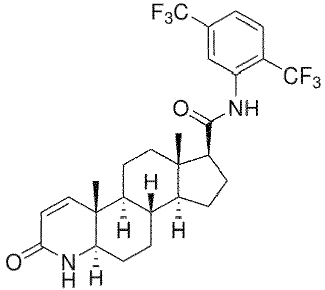

A pharmaceutical composition in the form of soft gelatin capsule comprising the active agent 17-beta-N-[2,5-bis(trifluoromethyl)]phenylcarbamoyl-4-aza-5-alpha-androst-1-en-3-one which can be stored at a temperature higher than 30 °C has not been marketed yet.

It is generally known that a capsule filled with a solution usually has a limited stability and a relatively short shelf life period. Study No. ARI10018 found that in earlier conventional dissolution testing of GI 198745 (dutasteride) soft gelatin capsules, a problem with cross-linking of gelatin was discovered, resulting in a decreased solubility of the gelatin and a reduced rate of drug release during in vitro testing and that these properties can reduce the stability of the dosage form, in particular under tropical conditions with elevated temperature and humidity. Furthermore, the manufacture of capsules containing a solution involves more complex processes than the manufacture of a solid form, in particular of a tablet.

WO9908666 (Glaxo Group Ltd) discloses soft capsules comprising dutasteride and a fatty acid ester of glycerol or propylene glycol. WO9908684 (Glaxo Group Ltd) discloses a pharmaceutical composition of a solution of active aza steroid with polyethylene glycol and propylene glycol and a soft gelatin capsule filled with the solution composition. Both prior-art patent applications are silent about the nature of the shell and how it affects the stability and the dissolution profiles of the soft gelatin capsules.

Dutasteride is practically insoluble in water (0.38 ng/ml). Hence, several approaches in the prior-art are based on improving its solubility and, as a consequence, its bioavailability by micronization of dutasteride and/or by dissolving dutasteride in solvents like oils. Accordingly, EP 2 050 436 (Siegfried Generics Int AG) describes a pharmaceutical composition of dutasteride, wherein due to micronization the average particle size of dutasteride ranges from 2 microns to 10 microns to achieve dissolution of more than 80% of dutasteride in a test medium within 10 minutes.

WO2010092596 (Genepharm India Private Limited) describes an oral pharmaceutical composition of dutasteride comprising one or more surfactant(s), one or more co-surfactant(s) and one or more oil(s). Upon dilution with aqueous medium the composition forms a microemulsion with at least 95% of the particles having a mean particle size below 200 nm. However, these formulations and in particular the SMEDDS (Self Microemulsifying Drug Delivery System) described in WO 2010092596 have several drawbacks. For example, relatively high concentrations of surfactant (s) and co-surfactant (s) are required. Surfactant (s) and co-surfactant (s) in high concentrations can modify the delivery of dutasteride in the gastrointestinal tract and the permeability of the active ingredient through the enterocytes. Moreover, the preparation of the composition includes several process steps. In addition, the composition exhibits stability problems with respect to hygroscopicity and sensitivity to oxygen. Also soft gelatin capsules are very sensitive to heat and humidity. In hot or humid climates, soft gelatin capsules may stick together or even break open before there is a chance to use them.

Consequently, there is still a need for a pharmaceutical composition of 17-beta-N-[2,5-bis(trifluoromethyl)phenyl]carbamoyl-4-aza-5-alpha-androst-1-en-3-one or a salt or solvate thereof which avoids the above mentioned disadvantages of the compositions of the prior art. In particular, a safe pharmaceutical composition of 17-beta-N-[2,5-bis(trifluoromethyl)]phenylcarbamoyl-4-aza-5-alpha-androst-1-en-3-one exhibiting a good stability and providing a good bioavailability of the active ingredient would be desirable. Thus, a pharmaceutical composition suitable for being stored up to 40 °C. Preferably, the composition should be obtainable by a simple and quick process.

Tamsulosin is the generic name for 5-[2-[[2-(2-ethoxyphenoxy)ethyl]amino] propyl]-2-methoxy-benzenesulfonamide and is described in US 4,703,063 and US 4,731,478. As described in the above-mentioned patents, tamsulosin has alpha-adrenergic blocking activity and is believed useful, inter alia, in the treatment of benign prostatic hyperplasia ("BPH"). Tamsulosin hydrochloride is marketed under various trade names, including Flomax®, Harnal®, and Omnic®, for the treatment of symptoms of BPH, such as urinary volume and frequency problems. US 4,772,475 describes a controlled-release pharmaceutical dosage form containing tamsulosin.

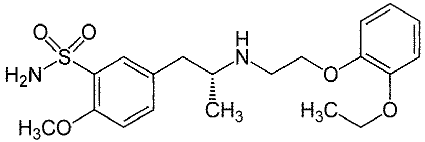

It is known that the active agent 17-beta-N-[2,5-bis(trifluoromethyl)] phenylcarbamoyl-4-aza-5-alpha-androst-1-en-3-one can be co-administered with tamsulosin in a medical treatment, e.g. in a treatment of benign prostatic hyperplasia. WO 2003090753 suggests the possibility of a combination of tamsulosin and finasteride or dutasteride, however it does not provide a particularly preferred technical solution to how the fixed combination may be formulated. WO 2006055659 suggests a fixed dose composition (FDC) of dutasteride and tamsulosin, wherein dutasteride is formulated in a soft gel and tamsulosin is formulated in form of beads, said beads comprising a multilayer composition with tamsulosin incorporated in one of these layers. Apparently, making a multilayer bead having reliable release rate of tamsulosin during its pathway in body fluids is technologically quite difficult and a simplification of the dosage form would be desirable. Both applications are silent in respect of the size of the soft gelatin capsule and the amount of the fill used while maintaining the same dose and bioavailability of the product.

The marketed product Duodart® is also a "capsule-in-capsule" formulation; it has a relatively short shelf life of 2 years with storage of the product below 30 °C. A further problem relates to the size of the combination capsule which does not allow easy swallowing by all patients in need of treatment for BPH.

There is still a need to provide an improved dosage form which would comprise an inner capsule loaded with the active agent which is to be co-administered with tamsulosin in form of pellets, granules or powder, and an outer capsule entirely covering the inner one, and to place the tamsulosin-containing coated pellets or granules or powder into the space between the inner and outer capsule. Then, after the administration, tamsulosin composition and the active agent are independently liberated from the capsules and they interact with body fluids in their own therapeutically effective ways.

A suitable technical solution of how to adapt the size and composition of dutasteride soft gelatin capsules to fulfil the above requirement has not been addressed in the prior art and thus there is a need for such a solution. An improvement to simplify treatment regimens for BPH and other diseases or conditions affected by 5-alpha reductase inhibition and/or alpha-adrenergic blocking would enhance patient compliance by providing a simplified dosage form containing pharmaceutically acceptable amounts of the two treatments.

The above issues have been addressed in the present invention and specific embodiments thereof.

### DESCRIPTION OF THE INVENTION

The present invention provides a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, which possess advantageous stable dissolution profiles and additionally disintegrates rapidly in aqueous solutions. Furthermore, the pharmaceutical composition in the form of an immediate release soft gelatin capsule of the present invention avoids the dissolution problems of the soft gelatin shell that become apparent upon aging and which are attributed to the cross-linking of gelatin. Thus, the pharmaceutical composition in the form of an immediate release soft gelatin capsule of the present invention avoids chemical, physical and dissolution instability, which would result in a slower dissolution profile with an incomplete release of the active ingredient.

High humidity causes the capsules to become soft, tacky, and bloated and may increase the likelihood of moisture migration from the shell into the fill material. Such a transfer can cause chemical, physical and dissolution instability.

Another advantage of the soft gelatin capsules as herein disclosed is that they are homogeneously filled and safe to handle. The soft gelatin capsules as herein disclosed can be manufactured by highly reproducible filling process, which helps ensure each soft gelatin capsule has the same drug content. Moreover, the soft gelatin capsules as herein disclosed shows good characteristics to be marketed.

In a first aspect of the first invention, it is provided a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; and wherein the shell of the soft gelatin capsule comprises an acid gelatin, preferably an acid gelatin having less than 150 g Bloom of gel strength; or a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the pharmaceutical composition is suitable for being stored up to 40 °C.

In a second aspect of the first invention, it is provided a pharmaceutical batch of at least 100,000 soft gelatin capsules of the pharmaceutical composition of the first aspect.

In a third aspect of the first invention, it is provided a blister comprising at least a unit dosage form of a pharmaceutical composition of the first aspect of the first invention.

In a fourth aspect of the first invention, it is provided a cardboard box with patient information leaflet comprising at least a blister with at least 4 soft gelatin capsules as defined in the pharmaceutical composition of the first aspect of the first invention.

In a fifth aspect of the first invention, it is provided the use of an acid gelatin for the manufacture of a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one.

In a sixth aspect of the first invention, it is provided a pharmaceutical composition in the form of a hard shell capsule comprising an immediate release soft gelatin capsule comprising the active agent 17-beta-N-[2,5-bis(trifluoromethyl)]phenylcarbamoyl-4-aza-5-alpha-androst-1-en-3-one as defined in the first aspect of the first invention and tamsulosin in the form of pellets or granules or powder..

In a further embodiment, it is provided a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; and wherein the shell of the soft gelatin capsule comprises an acid gelatin, preferably an acid gelatin having less than 150 g Bloom of gel strength; and wherein the pharmaceutical composition is suitable for being stored up to 40 °C.

In a further embodiment of the pharmaceutical composition as herein disclosed, the pharmaceutical composition is stable up to 40 °C, preferably is stable up to 40 °C at 75 % RH.

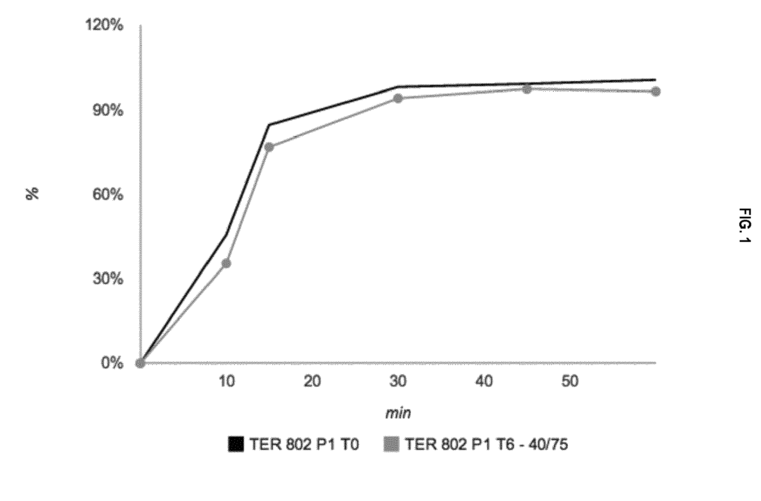

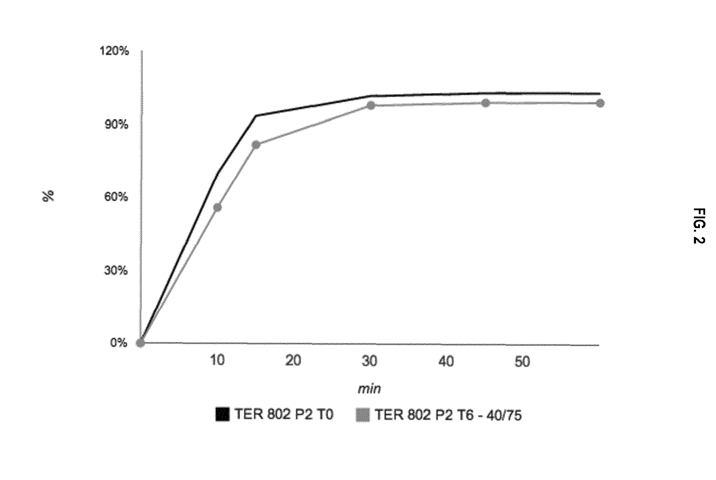

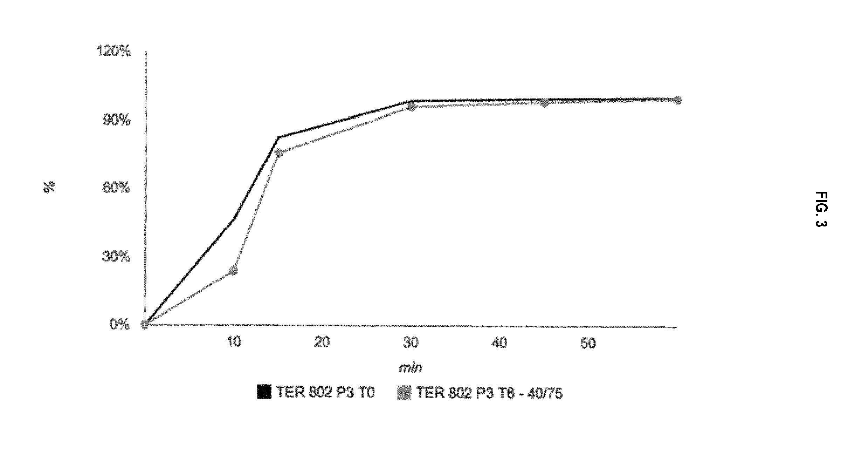

The soft gelatin capsules as herein disclosed are immediate release dosage forms. An immediate release dosage form as herein disclosed has to be understood as a dosage form having a dissolution performance such as 60 % or more of 17-beta-N-[2,5-bis(trifluoromethyl)]phenylcarbamoyl-4-aza-5-alpha-androst-1-en-3-one contained in said pharmaceutical composition dissolves within 60 minutes. In a preferred embodiment, the immediate release composition as herein disclosed releases at least 80 % of 17-beta-N-[2,5-bis(trifluoromethyl)]phenylcarbamoyl-4-aza-5-alpha-androst-1-en-3-one in 60 minutes. In another embodiment, the soft gelatin capsules as herein disclosed releases at least 80 % in 45 min, preferably in 35 min and more preferably in 30 min. In another embodiment, the soft gelatin capsules as herein disclosed releases at least 80 % in 15 min, and at least 95 % in 60 min. In another embodiment, the soft gelatin capsules as herein disclosed releases at least 80 % in 30 min and at least 95 % in 60 min. Figure 1, 2, 3 and 4 depict the immediate release dissolution profiles of a soft gelatin capsule which would be within some embodiments of the present invention. The dissolution test for an immediate release soft gelatin capsule comprising 17-beta-N-[2,5-bis(trifluoromethyl)]phenylcarbamoyl-4-aza-5-alpha-androst-1-en-3-one as herein disclosed is performed in the following conditions: USP Apparatus: II (Paddles). Speed: 50 r.p.m. Medium: 0.1N HCl, containing 2 % sodium lauryl sulfate. Wavelength 210 nm.

The immediate release soft gelatin capsules as herein disclosed are stable immediate release soft gelatin capsules. As used herein, the term "stable" refers to a soft gelatin capsules wherein the total content of impurities originating from the decomposition of 17-beta-N-[2,5-bis(trifluoromethyl)]phenylcarbamoyl-4-aza-5-alpha-androst-1-en-3-one does not exceed 5 % area, preferably 4 % area, more preferably 3 % area and most preferably 2 % area determined by liquid chromatography at 210 nm if such a composition is stored for 3 months at 30 °C, preferably 6 months at 40 °C / 75 % relative humidity (RH). Furthermore, the dissolution profiles of the soft gelatin capsules as herein disclosed when the soft gelatin capsules are stored for 3 months at 30 °C, preferably 6 months at 40 °C / 75 %RH still correspond to those of an immediate release composition, therefore the soft gelatin capsules as herein disclosed are suitable for being stored in such conditions.

In a further embodiment of the pharmaceutical composition as herein disclosed, the fill of the soft gelatin capsule further comprises at least a pharmaceutically acceptable excipient, such as glycerol monocaprylocaprate and butylhydroxytoluene, preferably the fill of the soft gelatin capsule further comprises glycerol monocaprylocaprate.

In a further embodiment of the pharmaceutical composition as herein disclosed, the fill of the soft gelatin capsule further comprises glycerol monocaprylocaprate and an antioxidant, preferably butylhydroxytoluene, preferably the fill of the soft gelatin capsule consists of the active agent 17-beta-N-[2,5-bis(trifluoromethyl)]phenylcarbamoyl-4-aza-5-alpha-androst-1-en-3-one, glycerol monocaprylocaprate and an antioxidant, preferably butylhydroxytoluene.

The esters useful in this invention preferably are derived from carboxylic acids containing from 6 to 12 carbon atoms. Particularly preferred are those esters derived from caprylic acid (8 carbon atoms). While mono, di, and tri-esters are all useful in this invention, monoesters are preferred. In addition, the ester may be part of a mixture comprising differing carbon atom content in the esters and/or comprising a mixture of monoglycerides, diglycerides, and triglycerides.

Commercially available esters are often such mixtures. For example glycerol monocaprylocaprate (also named as mono and diglycerides of caprylic/capric acid) is a mixture of monoacylglycerols, mainly mono-O-octanoylglycerol and mono-O-decanoylglycerol, containing variable quantities of di- and triacylglycerols, obtained by direct esterification of glycerol with caprylic (octanoic) and capric (decanoic) acids, followed by a distillation step in the case of glycerol monocaprylocaprate (type II). Content:
- glycerol monocaprylocaprate (type I): monoacylglycerols: 45.0 % to 75.0 %; diacylglycerols: 20.0 % to 50.0 %; triacylglycerols: maximum 10.0 %;
- glycerol monocaprylocaprate (type II): monoacylglycerols: minimum 80.0 %; diacylglycerols: maximum 20.0 %; triacylglycerols: maximum 5.0 %. (all percentages are weight percents). More information available in the European Pharmacopoeia 7th edition.

Also, some of these steroids are prone to oxidation. Gelatin capsule formulations can be much more resistant to oxidation due to the low oxygen permeation of typical gelatin shells. See, for example, F.S. Home et al., Soft Gelatin capsules II: Oxygen Permeability Study of Capsule Shells, J. Pharm. Schi., Vol. 64 (N° 5), 851-887 (1975).

It may also be useful to include an antioxidant in the composition. Suitable antioxidants include butylhydroxytoluene (BHT), butylhydroxyanisole (BHA), and ascorbic acid. A particularly preferred antioxidant is butylhydroxytoluene. Antioxidants may be used alone or in combination. The antioxidant or mixture of antioxidants is preferably from 0.001 to 0.5% by weight of the composition of this invention.

The shell of the soft gelatin capsule generally comprises gelatin, a plastifying agent and water therein as the main components. Accordingly, the soft gelatin capsule can be normally kept in satisfactory conditions, so far as the water content of the shell is maintained within a certain range. The water content is preferably maintained in the range from about 5 to about 10 % for shell of a soft capsule, more preferably from about 5 to about 8% for shell of a soft capsule. However, the water content of the soft capsule is apt to vary depending upon the circumferential conditions to deviate from the above-mentioned preferred range in the-course of lapse of time. If the water content reaches to so high level as to exceed the upper limit of the preferred range, the shell becomes wet to soften. Moreover, the soft capsules are apt to stick to each other to form an aggregated mass. On the other hand, if the water content reaches to a low level below the above-mentioned lower limit, the shell hardens to produce cracks therein. The soft capsule under these conditions shows poor lubricity and glidability so that the operations for packing the capsules cannot be carried out smoothly.

The determination of water content is measured by the method described in European Pharmacopoeia 7.0th edition 2.5.12 (Water: semi-micro determination). The semi-micro determination of water, also named as Karl Fischer titration, is based upon the quantitative reaction of water with sulfur dioxide and iodine in a suitable anhydrous medium in the presence of a base with sufficient buffering capacity.

Soft gelatin capsules are manufactured using a gelatin solution that is plasticized, preferably with propylene glycol, sorbitol, glycerol or various approved mixtures thereof. Soft gelatin capsules as herein disclosed can be manufacture-formed, filled and sealed in one continuous operation.

In a further embodiment of the pharmaceutical composition as herein disclosed, the shell of the soft gelatin capsule consists essentially of an acid gelatin.

In a further embodiment of the pharmaceutical composition as herein disclosed, the gelatin is an acid gelatin having from 75 to 145 g Bloom of gel strength, preferably from 85 to 135 g Bloom of gel strength, more preferably from 95 to 125 g Bloom of gel strength.

Among the different kinds of gelatins, there are A- (or acid) type and B- (or limed/alkaline) type. This categorisation essentially goes back to the pre-treatment of the raw material which will affect the characteristics of the gelatin extracted. Typical differences are the iso-electrical point as well as the viscosity in solution.

Gel strength and viscosity are the two most important measurements used to assess the grade and quality of a gelatin. The gel strength is determined using a texture analyzer. A pipet viscometer is used to determine viscosity. Both tests are based on a standard 6.67 % test solution. Gelatin weights for standard tests are made as is, without moisture correction.

Gel strength is expressed in (gram) Bloom. Commercial gelatins may vary from low Bloom (<150) medium Bloom (150 - 220) to high Bloom (> 220) types. The test was originally developed and patented in 1925 by O. T. Bloom. The procedure for gel strength determination is summarized as follows: A water solution consisting of 6.67 % gelatin (7.50 ± 0.1g gelatin and 105.0 ± 0.2g deionized water, melted at 60-65°C) is carefully prepared in a specified 150 ml, wide-mouth, glass bottle, which is then placed in a chilled water bath and held at 10 ± 0.1 °C for 17 ± 1 hours. After chilling, the rigidity of the gel is measured as the force, in grams, required to impress a standard 0.500 ± 0.001 inch diameter plunger to a depth of 4 millimeters into the surface of the gel. This weight is referred to as the gel strength, or Bloom rating, of the gelatin. The greater the force required, the higher the strength of the gel. Commercial gelatins range from 50 to 300 Bloom grams.

In practice, the viscosity of gelatin is usually measured on the very same sample used for gel strength determination. The 6.67% test solution is carefully brought to 60°C whereupon 100 ml is introduced into a calibrated capillary pipet. The efflux time, in seconds, is recorded, and later converted to millipoise based on the relationship established at time of calibration. Each individual gelatin viscosity pipet is calibrated with oils traceable to the National Institute for Standards and Technology. Molecular weight distribution appears to play a more important role in the effect on viscosity than it does on gel strength. Some gelatins of higher gel strength may have lower viscosities than gelatins of lower gel strength. The viscosity of gelatin solutions increases with increasing gelatin concentration and with decreasing temperature; viscosity is at a minimum at the isoionic point.

Gelatin is generally categorized into mesh sizes 5 to 100 mesh which translates to a U.S. STD sieve size. Gelatin that is 5 mesh would look like a piece of rice where 100 mesh would look like baking flour a fine white powder. The mesh or particle size does not affect the properties of the gelatin once it is dissolved.

In a further embodiment of the pharmaceutical composition as herein disclosed, the fill is from about 35 to about 85 % w/w of the total amount of the immediate release soft gelatin capsule and the shell is from about 15 to about 65 % w/w of the total amount of the immediate release soft gelatin capsule; preferably the fill is from about 45 to about 75 % w/w of the total amount of the immediate release soft gelatin capsule and the shell is from about 25 to about 55 % w/w of the total amount of the immediate release soft gelatin capsule; more preferably the fill is from about 55 to about 65 % w/w of the total amount of the immediate release soft gelatin capsule and the shell is from about 35 to about 45 % w/w of the total amount of the immediate release soft gelatin capsule.

In a further embodiment of the pharmaceutical composition as herein disclosed, the amount of fill is from 150 mg to 400 mg, preferably from 200 mg to 350 mg, more preferably from 235 mg to 315 mg of the total amount of the immediate release soft gelatin capsule.

The filling mixture is the main part of the fill of the stable immediate release soft gelatin capsule.

In a further embodiment of the pharmaceutical composition as herein disclosed, the amount of the active agent 17-beta-N-[2,5-bis(trifluoromethyl)]phenylcarbamoyl-4-aza-5-alpha-androst-1-en-3-one is from 0.4 to 0.6 mg of the total amount of the immediate release soft gelatin capsule, preferably 0.5 mg of the total amount of the immediate release soft gelatin capsule.

In a further embodiment of the pharmaceutical composition as herein disclosed, the hardness of the immediate release soft gelatin capsule is from 5.0 to 15.0 N, preferably from 7.0 and 13.0 N, more preferably from 9.0 and 11.0N. As used herein, the term "hardness" refers to the strength needed to move 2.0 mm of the surface of a soft gelatin capsule. Durometer is one of several measures of the hardness of a material, typically used as a measure of hardness in polymers, elastomers, and rubbers (e.g. Durometer Bareiss G7394-A or similar). Durometer, like many other hardness tests, measures the depth of an indentation in the material created by a given force on a standardized presser foot. The capsules are compressed to a certain extent between a measuring detector and a slowly moving plate. Under these test conditions, an optimum strength range is specified. Strength values above or below this specified range are indicative of insufficient flexibility or softening, respectively.

In a further embodiment of the pharmaceutical composition as herein disclosed, it is provided a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; wherein the shell of the soft gelatin capsule comprises an acid gelatin having from 80 to 145 g Bloom of gel strength; wherein the fill is from about 35 to about 85 % w/w of the total amount of the immediate release soft gelatin capsule and the shell is from about 15 to about 65 % w/w of the total amount of the immediate release soft gelatin capsule; wherein the amount of fill is from 150 mg to 400 mg of the total amount of the immediate release soft gelatin capsule and wherein the amount of the active agent 17-beta-N-[2,5-bis(trifluoromethyl)]phenylcarbamoyl-4-aza-5-alpha-androst-1-en-3-one is from 0.4 to 0.6 mg of the total amount of the immediate release soft gelatin capsule, preferably 0.5 mg of the total amount of the immediate release soft gelatin capsule.

In a further embodiment of the pharmaceutical composition as herein disclosed, it is provided a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the pharmaceutical composition is suitable for being stored up to 40 °C; wherein the gelatin is an acid gelatin having from 80 to 145 g Bloom of gel strength, the fill is from about 35 to about 85 % w/w of the total amount of the immediate release soft gelatin capsule and the shell is from about 15 to about 65 % w/w of the total amount of the immediate release soft gelatin capsule; wherein the amount of fill is from 150 mg to 400 mg of the total amount of the immediate release soft gelatin capsule and wherein the amount of the active agent 17-beta-N-[2,5-bis(trifluoromethyl)]phenylcarbamoyl-4-aza-5-alpha-androst-1-en-3-one is from 0.4 to 0.6 mg of the total amount of the immediate release soft gelatin capsule, preferably 0.5 mg of the total amount of the immediate release soft gelatin capsule.

In a further embodiment of the pharmaceutical batch as herein disclosed, the amount of 17-beta-N-[2,5-bis(trifluoromethyl)]phenylcarbamoyl-4-aza-5-alpha-androst-1-en-3-one in each soft gelatin capsule is from 0.4 to 0.6 mg.

In a further embodiment of the pharmaceutical batch as herein disclosed, the relative standard deviation (RSD) value of content uniformity is less than 5% w/w, preferably less than 1% w/w.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

Each pharmaceutical batch of finished product must fulfil the regulatory requirements of the corresponding Medicine Agency before being released for sale or supply, such as impurities thresholds and stability data.

The pharmaceutical composition may be manufactured at laboratory scale, not necessarily following GMP and not intended for commercialization. The pharmaceutical composition may also be manufactured for validation, following GMP. A batch of a pharmaceutical composition that is manufactured for validation is called "pilot batch".

In a further embodiment of the blister as herein disclosed, the blister is an aluminium/PVC/PVDC blister.

The term "blister" or "bubble pack" refers to a sheet in a package construction with recesses designed to hold dosage forms. The sheet may be a plastic film, foil, or combination thereof. Normally, a blister is a product consisting of a flat structure in which blisters are formed, generally by means of a heating process or blowing air, into which the single elements to be packaged are inserted. The blisters are then hermetically sealed using flat strips of appropriate thermomoldable materials (plastics, aluminium, paper), which represent the frangible element through which it is then possible to remove the product. The primary component of a blister pack is a cavity or pocket made from a formable web, usually a thermoformed plastic. This usually has a backing of paperboard or a lidding seal of aluminium foil or plastic. An aluminium/PVC blister refers to a blister where the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermoformed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packaging pharmaceutical products are the assurance of product/packaging integrity (including shelf life) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blister line. There are two types of blister machine's design: rotary and flat-plate.

As used herein, the term "unit dose" or "unit dosage" refers to a physically discrete unit that contains a predetermined quantity of active ingredient calculated to produce a desired therapeutic effect. The unit dose or unit dosage may be in the form of a tablet, capsule, soft gelatin capsule, sachet, etc. referred to herein as a "unit dosage form."

In a further embodiment of the cardboard box as herein disclosed, the patient information leaflet does not contain any information which prevents to store the pharmaceutical product above 30 °C.

It is known that the active substance 17-beta-N-[2,5-bis(trifluoromethyl)]phenylcarbamoyl-4-aza-5-alpha-androst-1-en-3-one might be co-administered with tamsulosin in a medical treatment, e.g. in a treatment of benign prostatic hyperplasia.

WO2004/043449 of Synthon discloses a pharmaceutical pellet composition comprising tamsulosin as active ingredient and having an advantageous coating layer for obtaining an extended release profile. WO2006055659 suggests a fixed dose composition of dutasteride and tamsulosin, wherein dutasteride is formulated in a soft gel and tamsulosin is formulated in the form of beads, said beads comprising a multilayer composition with tamsulosin incorporated in one of these layers.

In a further embodiment of the pharmaceutical composition in the form of a hard capsule of the sixth aspect, the amount of the active agent 17-beta-N-[2,5-bis(trifluoromethyl)]phenylcarbamoyl-4-aza-5-alpha-androst-1-en-3-one is from 0.4 to 0.6 mg, preferably 0.5 mg of the total amount of the hard capsule and the amount of tamsulosin is from 0.3 to 0.5 mg, preferably 0.4 mg of the total amount of the hard capsule.

In a further embodiment of the pharmaceutical composition in the form of a hard capsule as herein disclosed, the average content of tamsulosin hydrochloride in the population of pellets is less than 0.15 % w/w calculated on a dry pellet basis, preferably is from 0.10 to 0.145 % w/w calculated on a dry pellet basis.

In a further embodiment of the pharmaceutical composition in the form of a hard capsule as herein disclosed, the average content of tamsulosin hydrochloride in the population of granules is less than 1.0 % calculated on a dry basis, preferably less than 0.4 to % w/w calculated on a dry basis, more preferably is from 0.15 to 0.35 % w/w calculated on a dry basis.

A second invention provides a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, which has a smaller size than the soft gelatin capsules of the prior art, whereas maintaining the stability, bioavailability and efficacy of the pharmaceutical composition.

The compositions of the second invention offer the additional benefit of increasing the concentration of solubilized 17-beta-N-[2,5-bis(trifluoromethyl)]phenylcarbamoyl-4-aza-5-alpha-androst-1-en-3-one per total fill volume, which permits smaller fill volumes to be used to deliver the same dosage of the drug. The loading of the fill in the respective soft gelatin capsules is so adjusted to obtain a dosage form of the active agent exhibiting a good stability and providing a good bioavailability of the active ingredient.

In particular, such a kind of pharmaceutical composition is advantageous since it would require a lower quantity of excipients. Preferably all this would result in both economic (cheaper cost of formulation, packaging, etc.) and will have advantage with patients with swallowing difficulties as elderly, enhancing the treatment adherence or compliance for these patients.

In a first aspect, the second invention relates to a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the weight ratio active agent:total weight of the fill of the soft gelatin capsule is less than 1:600. Preferably, the weight ratio active agent:total weight of the fill of the soft gelatin capsule is between 1:240 to 1:600, more preferably between 1:300 to 1:560, and even more preferably between 1:380 to 1:520.

In a further embodiment of the pharmaceutical composition of the second invention, the soft gelatin capsule comprises less than 300 mg of a mixture of monoacylglycerols. Preferably the soft gelatin capsule comprises from 120 to 300 mg of a mixture of monoacylglycerols, more preferably from 150 to 280 mg of a mixture of monoacylglycerols, and even more preferably from 190 to 260 mg of a mixture of monoacylglycerols.

In a further embodiment, the mixture of monoacylglycerols is glycerol monocaprylocaprate.

In a further embodiment, the shell of the soft gelatin capsule comprises an acid gelatin, preferably an acid gelatin having less than 150 g Bloom of gel strength , preferably having from 75 to 145 g Bloom of gel strength, more preferably from 85 to 135 g Bloom of gel strength, and even more preferably from 95 to 125 g Bloom of gel strength.

In a further embodiment, the fill of the soft gelatin capsule further comprises glycerol monocaprylocaprate and at least an antioxidant, preferably butylhydroxytoluene. More preferably the fill of the soft gelatin capsule consists of the active agent 17-beta-N-[2,5-bis(trifluoromethyl)]phenylcarbamoyl-4-aza-5-alpha-androst-1-en-3-one, glycerol monocaprylocaprate and an antioxidant, preferably butylhydroxytoluene.

The second invention relates also to an improved fixed-dose combination of dutasteride and tamsulosin. The soft gelatin capsules of the first aspect of the second invention may be formulated into dosage units for coadministration of dutasteride with tamsulosin within a fixed dose combination. A suitable dosage unit is, for instance, an outer hard shell capsule, in which there is placed an inner soft gelatin capsule loaded with a composition of dutasteride which is to be co-administered with tamsulosin.

In a second aspect, the second invention relates to a pharmaceutical composition in the form of a hard shell capsule comprising an immediate release soft gelatin capsule comprising the active agent 17-beta-N-[2,5-bis(trifluoromethyl)]phenylcarbamoyl-4-aza-5-alpha-androst-1-en-3-one of the first aspect and tamsulosin in the form of pellets or granules or powder. Preferably, the amount of the active agent 17-beta-N-[2,5-bis(trifluoromethyl)]phenylcarbamoyl-4-aza-5-alpha-androst-1-en-3-one in the soft gelatin capsule of the first aspect of the second invention is from 0.4 to 0.6 mg, more preferably is 0.5 mg and the amount of tamsulosin is from 0.3 to 0.5 mg, and even more preferably is 0.4 mg.

The pellets or beads of the invention contain tamsulosin or a pharmaceutically acceptable salt thereof as active ingredient and advantageously are manufactured in homogeneous form having an enteric coating, as known from the state of the art. Thus, the pellets proposed herein, in essence, comprise: a) a core comprising tamsulosin and b) an outer coating layer surrounding the core, which comprises a pharmaceutically acceptable acid-resistant acrylic polymer. The pellets of tamsulosin used are well known in the art, see for further reference EP1562573, WO2010066268 and WO2004056354.

A suitable pellet that may be also used in the present invention are the tamsulosin pellets disclosed in WO2006055659, which are a plurality of pellets or beads manufactured in multilayer form comprising an inner inert core, on which a layer comprising tamsulosin and carrier is layered.

The term "pellet" or "bead" refers to a formulation that has been made by layering onto non-pareils or extrusion optionally followed by spheronization or other similar known techniques.

In a further embodiment, the average content of tamsulosin hydrochloride in the population of pellets is less than 0.15 % w/w calculated on a dry pellet basis, preferably is from 0.10 to 0.145 % w/w calculated on a dry pellet basis.

In a further embodiment, the tamsulosin in form of pellets exhibits a dissolution release profile in simulated gastric fluid using Ph. Eur. paddles method at 50 rpm which includes releasing less than 25% of the tamsulosin during the first two hours.

In a further embodiment of the pharmaceutical composition as herein disclosed, the tamsulosin in form of pellets exhibits a dissolution release profile in a phosphate buffer of pH 6.8 using Ph. Eur. paddles method at 100 rpm which includes releasing 15-45% of the tamsulosin in 30 minutes.

The pellets of the present invention preferably exhibit a dissolution release profile, wherein less than 25 % of tamsulosin, preferably less than 15% of tamsulosin and most preferably less than 10% of tamsulosin is released during the first two hours in simulated gastric fluid using Ph. Eur. paddles method at 50 rpm. Accordingly, once the coated pellets of the present invention are ingested, tamsulosin is released into the body at a rate that is characterized by minimizing the release during the pellets' residence time in the stomach environment. More advantageously, the pellet core size and composition as well as the material and the relative amount of the coating are selected so that the resulting population of pellets exhibits at least one of the following release rates in simulated intestinal fluid (sometimes referred to herein as phosphate buffer of pH 6.8), using Ph. Eur. paddles method at 100 rpm: 30-65%, preferably 40-60 % of the tamsulosin in one hour, and/or more than 80% of the tamsulosin in six hours. More preferably, the pellets satisfy all two release rates.

The granules of the invention contain tamsulosin or a pharmaceutically acceptable salt thereof as active ingredient and advantageously have a homogeneous distribution of active ingredient through the choice of the carrier matrix, even if the active ingredient is present in only small amounts, as known from the state of the art. The combination of these components as carrier matrix makes it possible for the release of tamsulosin to be time-and pH dependent and thus for the release profile *in vivo* to be optimal. Suitable granules that may be used in the present invention are the tamsulosin granules disclosed in WO2006005512, which are granules for controlled release of tamsulosin manufactured by using a standard granulation technique using a high speed mixer and the granules include a carrier matrix.

The term "pharmaceutical granulate" or "granulate" or "granular component" as used herein refers to the part of the pharmaceutical composition that has been obtained by granulating a powder into larger particles herein called granules.

In a further embodiment, the average content of tamsulosin hydrochloride in the population of granules is less than 1.0 % calculated on a dry basis, preferably less than 0.4 % w/w calculated on a dry basis, more preferably is from 0.15 to 0.35 % w/w calculated on a dry basis.

In a further embodiment, the tamsulosin in form of granules exhibits a dissolution release profile wherein between 15 and 30 % of tamsulosin is released from the granules in 60 minutes in phosphate buffer of pH 6.8, using Ph. Eur. basket method at 50 rpm.

The pellets of the present invention preferably exhibit a dissolution release profile, wherein more than 20 % of tamsulosin, preferably more than 30% of tamsulosin and most preferably more than 40% of tamsulosin is released during the first two hours in phosphate buffer of pH 6.8, using Ph. Eur. basket method at 50 rpm.

In a further embodiment, the total volume of the hard shell capsule is equal or less than 1 ml, preferably from 0.6 ml to 0.95 ml, more preferably from 0.7 to 0.9 ml.

In a preferred embodiment, the size of hard shell capsule is selected from 00 or 0 elongated, more preferably it is 0 elongated.

The hard shell capsules of a suitable size may be made, e.g., from hard gelatin or hydroxypropyl methylcellulose. Preferably, the hard shell capsules are hard hydroxypropyl methylcellulose capsules (HPMC capsules). Alternative, they may be made of hard gelatin.

In a further embodiment, the total volume of the soft gelatin capsule is equal or less than 0.4 ml, preferably from 0.2 to 0.37 ml, more preferably from 0.25 to 0.31 ml.

In a preferred embodiment, the size of the soft gelatin capsule is selected from 5 oblong, 4 oblong or 3 oblong, more preferably 4 oblong. The most preferred size is 4 oblong.

In a third aspect, the second invention relates to a pharmaceutical batch of at least 100,000 soft gelatin capsules of the pharmaceutical composition of the first aspect of the second invention. Preferably, the amount of 17-beta-N-[2,5-bis(trifluoromethyl)]phenylcarbamoyl-4-aza-5-alpha-androst-1-en-3-one in each soft gelatin capsule is from 0.4 to 0.6 mg.

Another aspect of the second invention relates to a blister comprising at least a unit dosage form of a pharmaceutical composition in the form of soft gelatin capsule of the first aspect of the second invention. Preferably, the blister is an aluminium/PVC/PVDC blister.

A third invention provides a pharmaceutical composition in the form of a hard shell capsule comprising tamsulosin in the form of pellets or granules or powder and an immediate release soft gelatin capsule comprising the active agent 17-beta-N-[2,5-bis(trifluoromethyl)]phenylcarbamoyl-4-aza-5-alpha-androst-1-en-3-one.

In a first aspect, the third invention relates to a pharmaceutical composition in the form of a hard shell capsule comprising:
(a) an immediate release soft gelatin capsule comprising the active agent 17-beta-N-[2,5-bis(trifluoromethyl)]phenylcarbamoyl-4-aza-5-alpha-androst-1-en-3-one, and
(b) tamsulosin in the form of pellets, wherein the average content of tamsulosin hydrochloride in the population of pellets is less than 0.15 to % w/w calculated on a dry pellet basis. Preferably, the average content of tamsulosin hydrochloride in the population of pellets is from 0.10 to 0.145 % w/w calculated on a dry pellet basis.

In another aspect, the third invention relates to a pharmaceutical composition in the form of a hard shell capsule comprising:
(a) an immediate release soft gelatin capsule comprising the active agent 17-beta-N-[2,5-bis(trifluoromethyl)]phenylcarbamoyl-4-aza-5-alpha-androst-1-en-3-one and
(b) tamsulosin in the form of granules, wherein the average content of tamsulosin hydrochloride in the population of granules is less than 1.0 % calculated on a dry basis, preferably less than 0.4 % w/w calculated on a dry basis. More preferably, the average content of tamsulosin hydrochloride in the population of pellets is from 0.15 to 0.35 % w/w calculated on a dry basis.

In a further embodiment of the pharmaceutical composition of the third invention, the amount of the active agent 17-beta-N-[2,5-bis(trifluoromethyl)]phenylcarbamoyl-4-aza-5-alpha-androst-1-en-3-one is from 0.4 to 0.6 mg, preferably is 0.5 mg and the amount of tamsulosin is from 0.3 to 0.5 mg, preferably is 0.4 mg.

The compositions of the third invention offer the additional benefit of being able to combine in a pharmaceutical composition a smaller size of any soft gelatin capsule and tamsulosin pellets with a certain average content of tamsulosin hydrochloride, which can be used to administer the drugs to patients, thereby increasing patient compliance, reducing manufacturing costs and obtaining a an environmentally friendly pharmaceutical composition due to less material used.

In a further embodiment, the weight ratio active agent:total weight of the fill of the soft gelatin capsule is less than 1:600, preferably the weight ratio is between 1:240 to 1:600, more preferably between 1:300 to 1:560, and even more preferably between 1:380 to 1:520.

In a further embodiment, the soft gelatin capsule comprises less than 300 mg of a mixture of monoacylglycerols, preferably the soft gelatin capsule comprises from 120 to 300 mg of a mixture of monoacylglycerols, more preferably from 150 to 280 mg of a mixture of monoacylglycerols, and even more preferably from 190 to 260 mg of a mixture of monoacylglycerols.

In a further embodiment, the mixture of monoacylglycerols is glycerol monocaprylocaprate.

In a further embodiment, the shell of the soft gelatin capsule comprises an acid gelatin, preferably an acid gelatin having less than 150 g Bloom of gel strength, preferably the gelatin is an acid gelatin having from 75 to 145 g Bloom of gel strength, more preferably from 85 to 135 g Bloom of gel strength, even more preferably from 95 to 125 g Bloom of gel strength.

Another aspect of the third invention relates to a pharmaceutical batch of at least 100,000 hard shell capsules of the pharmaceutical composition of the sixth aspect of the first invention, of the second aspect of the second invention or of the pharmaceutical compositions in the form of a hard shell capsule of the third invention. Preferably, the amount of 17-beta-N-[2,5-bis(trifluoromethyl)]phenylcarbamoyl-4-aza-5-alpha-androst-1-en-3-one in each hard capsule is from 0.4 to 0.6 mg and the amount of tamsulosin is from 0.3 to 0.5 mg.

Another aspect of the third invention relates to a high density polyethylene (HDPE) bottle comprising at least a unit dosage form of a pharmaceutical composition of the sixth aspect of the first invention, of the second aspect of the second invention or of the pharmaceutical compositions in the form of a hard shell capsule of the third invention. Preferably, the HPDE bottle has a polypropylene child-resistant closure with induction-seal liners.

Another aspect of the third invention relates to a cardboard box with patient information leaflet comprising at least an HPDE bottle with at least 4 hard capsules of a pharmaceutical composition of the sixth aspect of the first invention, of the second aspect of the second invention or of the pharmaceutical compositions in the form of a hard shell capsule of the third invention.

Another aspect of the third invention relates to the use of the pharmaceutical compositions described in any of the first, second or third inventions for the manufacture of a medicament for the treatment of the symptoms of benign prostatic hyperplasia.

A fourth invention provides processes for preparing pharmaceutical composition in the form of soft gelatin capsules. The processes of the present invention are characterized by improving the consistency and uniformity of the soft gelatin capsules and reducing the standard deviation of the soft gelatin capsule dimensions. The present invention reduces defects such as dimples and bubbles, provides a more uniform product which facilitates subsequent operations such as printing and blister packaging, and improves shelf life stability counteracting the effect of the stress caused by water loss during drying, gelatin shrinkage and material migration.

In a first aspect, the fourth invention relates to a process for manufacturing of soft gelatin capsules comprising the active agent 17-beta-N-[2,5-bis(trifluoromethyl)]phenylcarbamoyl-4-aza-5-alpha-androst-1-en-3-one comprising at least the following steps:
i) preparing a soft gelatin capsule fill solution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-one; wherein the active agent has a water content higher than 0.05 % w/w;
ii) encapsulating the fill obtained in step (i) in a shell to form filled soft gelatin capsules and;
iii) optionally drying the soft gelatin capsules;
preferably, wherein the temperature of the fill of steps (i) and (ii) is independently less than 40 °C.

Another advantage of the process as herein disclosed is that it is highly reproducible and robust independently of the water content of the active agent. It has been found that when the active agent used for manufacturing the soft gelatin capsules of the present invention is anhydrous, the dissolution is not complete at the usual working range of temperatures of 20-25 °C. However, when the active agent has a water content higher than 0.05% measured by Karl Fischer titration, the components of the fill are completely dissolved at the same range of temperatures. Furthermore, the inventors have been able to establish an optimum range of working temperatures able to obtain a fill solution completely dissolved homogeneously with active agents with a water content higher than 0.05 % w/w.

For the manufacture of the soft gelatin capsules, glycerol monocaprylocaprate was heated to approximately 32-34 °C. Then, butylhydroxytoluene was added and the mixture was stirred until dissolved. Then, the active agent was added and mixed, and the temperature of the mixture was maintained and monitored to ensure that it did not exceed 40 °C, until dissolved. Optionally, the solution was deaerated prior to encapsulation.

In a further embodiment of the process of the first aspect of the fourth invention, the active agent 17-beta-N-[2,5-bis(trifluoromethyl)]phenylcarbamoyl-4-aza-5-alpha-androst-1-en-3-one has a water content from 0.06 to 4 % w/w, preferably from 0.1 to 3 % w/w.

In a further embodiment of the process of the first aspect of the fourth invention, the temperature of the fill of steps (i) and (ii) ranges independently between 20 to 35 °C.

In a further embodiment of the process of the first aspect as herein disclosed, the active agent 17-beta-N-[2,5-bis(trifluoromethyl)]phenylcarbamoyl-4-aza-5-alpha-androst-1-en-3-one has a water content from 0.1 to 0.8 % w/w and the temperature of the fill of steps (i) and (ii) ranges independently between 28 to 34 °C, preferably the temperature of the fill of steps (i) and (ii) ranges independently between 30 to 32 °C.

In a further embodiment of the process of the first aspect as herein disclosed, the active agent 17-beta-N-[2,5-bis(trifluoromethyl)]phenylcarbamoyl-4-aza-5-alpha-androst-1-en-3-one has a water content from 0.8 to 2.5 % w/w and the temperature of the fill of steps (i) and (ii) ranges independently between 23 to 30 °C, preferably the temperature of the fill of steps (i) and (ii) ranges independently between 25 to 28 °C.

In a further embodiment of the process of the first aspect as herein disclosed, step (ii) comprises encapsulating the soft gelatin capsule fill obtained in step (i) using a rotary die and producing the soft gelatin capsule.

In a further embodiment, the process of the first aspect of the fourth invention for manufacturing the pharmaceutical composition of the first aspect of the first invention, of the sixth aspect of the first invention, of the first aspect of the second invention, of the second aspect of the second invention or of the pharmaceutical compositions in the form of a hard shell capsule of the third invention.

A second aspect of the fourth invention relates to the pharmaceutical composition obtained by the process of the first aspect of the fourth invention.

The gelatin solution needed in the production of soft capsules is called gelatin melting. The gelatin was prepared by blending-glycerol and purified water. The resulting mixture was heated in a pressurized reactor to about 70 °C to about 90 °C. Then the gelatin was added into the tank under stirring conditions. The gelatin solution was deaerated and was then maintained in the molten state until used for encapsulation.

Encapsulation was performed using a rotary die process. The heated gelatin was fed to an encapsulation machine where it entered two spreader boxes which cast the gelatin on a cooling drum, thus forming two gelatin ribbons. Each gelatin ribbon was lubricated with a mixture of medium chain triglycerides, optionally comprising 0.1 % w/w of soy lecithin. The medium chain triglycerides prevent the gelatin from sticking to the equipment and the soy lecithin prevents the capsules from sticking together after manufacture, prior to drying. The ribbons were then conveyed to the encapsulation roller. Die cavities to form the capsules are located on the circumference of the two adjacent rollers, which rotate and pull the gelatin ribbons between them. The die is of the desired capsule shape and size and the capsule is formed at the point where the two rotating dies meet. The fill solution was injected, by a metered positive-displacement pump, between the gelatin ribbons forcing them to expand and fill the die cavities. As the capsules were filled, they were simultaneously shaped, sealed and cut from the gelatin ribbon by the encapsulation rollers. The capsules were then conveyed to the rotating basket dryer.

Surprisingly, the process of encapsulating the fill materials with the shell material used according to the invention have proven to be suitable for producing soft capsules which are filled with molten fill material. In particular, the shell materials used according to the invention can be subjected during the capsule production to specific temperatures resulting in a stable soft gelatin capsules which avoid the breakage of the soft gelatin capsules, tackiness and have good rheological properties. It has been found that when the temperature of the gelatin ribbons in the steps of forming said gelatin ribbons and encapsulating the fill in said gelatin ribbons to form filled soft gelatin capsules ranges between 35 to 50 °C, the breakage of the soft gelatin capsules and tackiness are avoided while good rheological properties are obtained.

The transfer of molten gelatin from a holding tank to the spreader box is achieved in one of two ways. A useful method is to suspend or mount the tank of molten gelatin above the encapsulation machine and allow the molten material to be fed via gravity through heated tubes into the reservoir of the spreader box. Another method used is to pump the molten gelatin via heated tubes from floor mounted gelatin staging tanks using either a peristaltic or lobe pump system. One disadvantage of the pump feed system is that the pump casing/components and in-line connections must be maintained above the melting point of the film-forming composition. If there are cold areas within the path, the material will solidify and prevent flow. In addition, both gravity and pump systems require a method of controlling flow to prevent overfilling of the spreader boxes.

This process also requires maintaining the gelatin melt in a molten state from initial manufacture to just before encapsulation. Tanks used to feed the encapsulation machine require the entire tank to be maintained above the melt temperature of the film-forming composition. Prolonged maintenance of gelatin or other film-forming compositions in a molten state leads to degradation of the polymer, rendering the gelatin after prolonged staging, ineffective at fabricating capsules.

It has been found that gravity systems result in a more reproducible and robust process and, furthermore, can avoid further problems in the final encapsulation step. With this system the molten gelatin temperature can be easily controlled and it can be kept within the established range.

The capsules were dried by tumbling in a rotating basket dryer to remove sufficient moisture to allow handling.

They were then transferred onto trays and allowed to dry until the suitable hardness.

In a third aspect, the fourth invention relates to a process for manufacturing a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; wherein the shell of the soft gelatin capsule comprises an acid gelatin, preferably an acid gelatin having less than 150 g Bloom of gel strength; comprising at least the following steps:
i) forming the gelatin ribbons;
ii) encapsulating the fill in the gelatin ribbons of step (i) to form filled soft gelatin capsules and;
iii) optionally drying the soft gelatin capsules;
wherein the temperature of the gelatin ribbons of step (i) and (ii) ranges between 35 to 50 °C.

In a further embodiment of the process of the third aspect as herein disclosed, the process of the third aspect of the fourth invention, the temperature of the gelatin ribbons of step (i) and (ii) ranges between 40 to 45 °C, preferably the temperature of the gelatin ribbons of step (i) and (ii) ranges between 41 to 44 °C.

In a further embodiment of the process of the third aspect as herein disclosed, the gelatin is an acid gelatin having from 75 to 145 g Bloom of gel strength, preferably from 85 to 135 g Bloom of gel strength, more preferably from 95 to 125 g Bloom of gel strength.

In a further embodiment of the process of the third aspect as herein disclosed, for manufacturing the pharmaceutical composition of the first aspect of the first invention, of the sixth aspect of the first invention, of the first aspect of the second invention, of the second aspect of the second invention or of the pharmaceutical compositions in the form of a hard shell capsule of the third invention.

A more detailed description of how to manufacture soft gelatin capsules may be found in Ebert, "Soft Elastic Gelatin Capsules: A Unique Dosage Form" Pharmaceutical Technology, October 1977 and in "The Theory and Practice of Industrial Pharmacy", Chapter 13, Lachman et al., published by Lea and Febiger, 1970.

A fourth aspect of the fourth invention relates to the pharmaceutical pharmaceutical composition obtained by the process of the third aspect of the fourth invention.

In a further embodiment of the pharmaceutical pharmaceutical composition obtained by the process of the third aspect of the fourth invention, the hardness of the immediate release soft gelatin capsules is from 5.0 to 15.0 N, preferably from 7.0 and 13.0 N, more preferably from 9.0 N and 11.0 N.

Another aspect of the fourth invention relates to a pharmaceutical batch of at least 100,000 soft gelatin capsules of the pharmaceutical composition of the second aspect of the fourth invention or of the fourth aspect of the fourth invention. Preferably, the amount of 17-beta-N-[2,5-bis(trifluoromethyl)]phenylcarbamoyl-4-aza-5-alpha-androst-1-en-3-one in each soft gelatin capsule is from 0.4 to 0.6 mg.

Another aspect of the fourth invention relates to a blister comprising at least a unit dosage form of a pharmaceutical composition of the second aspect of the fourth invention or of the fourth aspect of the fourth invention. Preferably, the blister is an aluminium/PVC/PVDC blister.

Another aspect of the fourth invention relates to a cardboard box with patient information leaflet comprising at least a blister with at least 4 soft gelatin capsules of a pharmaceutical composition of the first aspect of the second invention, of the second aspect of the fourth invention or of the fourth aspect of the fourth invention.

A preferred embodiment of the present invention relates to a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; wherein the shell of the soft gelatin capsule comprises an acid gelatin, preferably an acid gelatin having from 75 to 145 g Bloom of gel strength; wherein the weight ratio active agent:total weight of the fill is between 1:240 to 1:600; and wherein the process for the manufacturing of said soft gelatin capsules comprises at least the following steps:
i) preparing a soft gelatin capsule fill solution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-one; wherein the active agent has a water content higher than 0.05 % w/w;
ii) encapsulating the fill resulting in step (i) in a shell to form filled soft gelatin capsules and;
iii) optionally drying the soft gelatin capsules;
preferably, wherein the temperature of the fill of the steps (i) and (ii) ranges between 20 to 35 °C.

Another preferred embodiment of the present invention relates to a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; wherein the shell of the soft gelatin capsule comprises an acid gelatin, preferably an acid gelatin having from 75 to 145 g Bloom of gel strength; wherein the weight ratio active agent:total weight of the fill is between 1:240 to 1:600; and wherein the process for the manufacturing of said soft gelatin capsules comprises at least the following steps:
i) forming the gelatin ribbons;
ii) encapsulating the fill in the gelatin ribbons of step (i) to form filled soft gelatin capsules and;
iii) optionally drying the soft gelatin capsules;
wherein the temperature of the gelatin ribbons of step (i) and (ii) ranges between 35 to 50 °C. Another aspect of the fourth invention relates to the use of the pharmaceutical compositions described in any of the first, second, third or fourth inventions for the manufacture of a medicament for the treatment of the symptoms of benign prostatic hyperplasia.

Unless otherwise indicated, all the analysis methods are carried out according to the European Pharmacopoeia 7th edition.

Further aspects and embodiments of the present invention can be found in the following numbered clauses:
Clause 1.- A pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; and wherein the shell of the soft gelatin capsule comprises an acid gelatin, preferably an acid gelatin having less than 150 g Bloom of gel strength; or a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the pharmaceutical composition is suitable for being stored up to 40 °C.
Clause 2.- The pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; and wherein the shell of the soft gelatin capsule comprises an acid gelatin, preferably an acid gelatin having less than 150 g Bloom of gel strength; and wherein the pharmaceutical composition is suitable for being stored up to 40 °C.
Clause 3.- The pharmaceutical composition according to anyone of the preceding claims wherein the pharmaceutical composition is stable up to 40 °C.
Clause 4.- The pharmaceutical composition according to anyone of the preceding claims, wherein the pharmaceutical composition is stable up to 40 °C at 75 % RH.
Clause 5.- The pharmaceutical composition according to anyone of the preceding claims, wherein the pharmaceutical composition is stable up to 30 °C at 75 % RH.
Clause 6.- The pharmaceutical composition according to anyone of the preceding claims, wherein the fill of the soft gelatin capsule further comprises at least a pharmaceutically acceptable excipient, such as glycerol monocaprylocaprate and butylhydroxytoluene.
Clause 7.- The pharmaceutical composition according to anyone of the preceding claims, wherein the fill of the soft gelatin capsule further comprises glycerol monocaprylocaprate.
Clause 8.- The pharmaceutical composition according to anyone of the preceding claims, wherein the fill of the soft gelatin capsule further comprises glycerol monocaprylocaprate and at least an antioxidant, preferably butylhydroxytoluene.
Clause 9.- The pharmaceutical composition according to anyone of the preceding claims, wherein the fill of the soft gelatin capsule consists of the active agent 17-beta-N-[2,5-bis(trifluoromethyl)]phenylcarbamoyl-4-aza-5-alpha-androst-1-en-3-one, glycerol monocaprylocaprate and an antioxidant, preferably butylhydroxytoluene.
Clause 10.- The pharmaceutical composition according to anyone of the preceding claims, wherein the gelatin is an acid gelatin having from 75 to 145 g Bloom of gel strength.
Clause 11.-The pharmaceutical composition according to anyone of the preceding claims, wherein the gelatin is an acid gelatin having from 85 to 135 g Bloom of gel strength.
Clause 12.-The pharmaceutical composition according to anyone of the preceding claims, wherein the gelatin is an acid gelatin having from 95 to 125 g Bloom of gel strength.
Clause 13.-The pharmaceutical composition according to anyone of the preceding claims, wherein the fill is from about 35 to about 85 % w/w of the total amount of the immediate release soft gelatin capsule and the shell is from about 15 to about 65 % w/w of the total amount of the immediate release soft gelatin capsule.
Clause 14.- The pharmaceutical composition according to any one of the preceding claims, wherein the fill is from about 45 to about 75 % w/w of the total amount of the immediate release soft gelatin capsule and the shell is from about 25 to about 55 % w/w of the total amount of the immediate release soft gelatin capsule.
Clause 15.- The pharmaceutical composition according to any one of the preceding claims, wherein the fill is from about 55 to about 65 % w/w of the total amount of the immediate release soft gelatin capsule and the shell is from about 35 to about 45 % w/w of the total amount of the immediate release soft gelatin capsule.
Clause 16.-The pharmaceutical composition according to any one of the preceding claims, wherein the amount of fill is from 150 mg to 400 mg of the total amount of the immediate release soft gelatin capsule.
Clause 17.-The pharmaceutical composition according to any one of the preceding claims, wherein the amount of fill is from 200 mg to 350 mg of the total amount of the immediate release soft gelatin capsule.
Clause 18.-The pharmaceutical composition according to any one of the preceding claims, wherein the amount of fill is from 235 mg to 315 mg of the total amount of the immediate release soft gelatin capsule.
Clause 19.-The pharmaceutical composition according to any one of the preceding claims, wherein the amount of the active agent 17-beta-N-[2,5-bis(trifluoromethyl)]phenylcarbamoyl-4-aza-5-alpha-androst-1-en-3-one is from 0.4 to 0.6 mg of the total amount of the immediate release soft gelatin capsule, preferably 0.5 mg of the total amount of the immediate release soft gelatin capsule.
Clause 20.-The pharmaceutical composition according to any one of the preceding claims, wherein the hardness of the immediate release soft gelatin capsule is from 5.0 to 15.0 N, preferably from 7.0 and 13.0 N, more preferably from 9.0 N and 11.0 N.
Clause 21.-A pharmaceutical batch of at least 100,000 soft gelatin capsules of the pharmaceutical composition according to any one of the preceding product claims.
Clause 22.-The pharmaceutical batch according to the preceding claim, wherein the amount of 17-beta-N-[2,5-bis(trifluoromethyl)]phenylcarbamoyl-4-aza-5-alpha-androst-1-en-3-one in each soft gelatin capsule is from 0.4 to 0.6 mg.
Clause 23.-A blister comprising at least a unit dosage form of a pharmaceutical composition according to any one of the preceding claims.
Clause 24.- The blister comprising a unit dosage of the pharmaceutical composition according to the preceding claim, wherein the blister is an aluminium/PVC/PVDC blister.
Clause 25.- A cardboard box with patient information leaflet comprising at least a blister with at least 4 soft gelatin capsules as defined in any one of the preceding pharmaceutical composition claims.
Clause 26.- The cardboard box according to the preceding claim, wherein the patient information leaflet does not contain any information which prevents to store the pharmaceutical product above 30 °C.
Clause 27.- Use of an acid gelatin for the manufacture of a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one.
Clause 28.-A pharmaceutical composition in the form of a hard shell capsule comprising an immediate release soft gelatin capsule comprising the active agent 17-beta-N-[2,5-bis(trifluoromethyl)]phenylcarbamoyl-4-aza-5-alpha-androst-1-en-3-one according to anyone of the preceding claims and tamsulosin in the form of pellets or granules or powder.
Clause 29.-The pharmaceutical composition in the form of a hard shell capsule according to the preceding claim wherein the amount of the active agent 17-beta-N-[2,5-bis(trifluoromethyl)]phenylcarbamoyl-4-aza-5-alpha-androst-1-en-3-one is from 0.4 to 0.6 mg, preferably is 0.5 mg of the total amount of the hard capsule and the amount of tamsulosin is from 0.3 to 0.5 mg, preferably is 0.4 mg of the total amount of the hard capsule.
Clause 30.-The pharmaceutical composition in the form of a hard shell capsule according to the two preceding claims wherein the average content of tamsulosin hydrochloride in the population of pellets is less than 0.15 to % w/w calculated on a dry pellet basis, preferably is from 0.10 to 0.145 % w/w calculated on a dry pellet basis.
Clause 31.- The pharmaceutical composition in the form of a hard shell capsule according to any one of claims 28 or 29 wherein the average content of tamsulosin hydrochloride in the population of granules is less than 1.0 % calculated on a dry basis, preferably less than 0.4 to % w/w calculated on a dry basis, more preferably is from 0.15 to 0.35 % w/w calculated on a dry basis.
Clause 32.- A pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the weight ratio active agent:total weight of the fill of the soft gelatin capsule is less than 1:600.
Clause 33.-The pharmaceutical composition according to the preceding claim, wherein the weight ratio active agent:total weight of the fill is between 1:240 to 1:600, preferably between 1:300 to 1:560, more preferably between 1:380 to 1:520.
Clause 34.- The pharmaceutical composition according to any one of the two preceding claims, wherein the soft gelatin capsule comprises less than 300 mg of a mixture of monoacylglycerols.
Clause 35.-The pharmaceutical composition according to any one of the three preceding claims wherein the soft gelatin capsule comprises from 120 to 300 mg of a mixture of monoacylglycerols, preferably from 150 to 280 mg of a mixture of monoacylglycerols, more preferably from 190 to 260 mg of a mixture of monoacylglycerols.
Clause 36.-The pharmaceutical composition according to any one of the four preceding claims wherein the mixture of monoacylglycerols is glycerol monocaprylocaprate.
Clause 37.- The pharmaceutical composition according to any one of the five preceding claims wherein the shell of the soft gelatin capsule comprises an acid gelatin, preferably an acid gelatin having less than 150 g Bloom of gel strength.
Clause 38.-The pharmaceutical composition according to any one of the six preceding claims, wherein the gelatin is an acid gelatin having from 75 to 145 g Bloom of gel strength, preferably from 85 to 135 g Bloom of gel strength, more preferably from 95 to 125 g Bloom of gel strength.
Clause 39.-The pharmaceutical composition according to any one of the seven preceding claims, wherein the fill of the soft gelatin capsule further comprises glycerol monocaprylocaprate and at least an antioxidant, preferably butylhydroxytoluene.
Clause 40.-The pharmaceutical composition according to any one of the eight preceding claims, wherein the fill of the soft gelatin capsule consists of the active agent 17-beta-N-[2,5-bis(trifluoromethyl)]phenylcarbamoyl-4-aza-5-alpha-androst-1-en-3-one, glycerol monocaprylocaprate and an antioxidant, preferably butylhydroxytoluene.
Clause 41.-A pharmaceutical composition in the form of a hard shell capsule comprising an immediate release soft gelatin capsule comprising the active agent 17-beta-N-[2,5-bis(trifluoromethyl)]phenylcarbamoyl-4-aza-5-alpha-androst-1-en-3-one according to anyone of the claims 32 to 40 and tamsulosin in the form of pellets or granules or powder.
Clause 42.- The pharmaceutical composition in the form of a hard shell capsule according to the preceding claim, wherein the amount of the active agent 17-beta-N-[2,5-bis(trifluoromethyl)]phenylcarbamoyl-4-aza-5-alpha-androst-1-en-3-one is from 0.4 to 0.6 mg, preferably is 0.5 mg and the amount of tamsulosin is from 0.3 to 0.5 mg, preferably is 0.4 mg.
Clause 43.-The pharmaceutical composition in the form of a hard shell capsule according to any one of the two preceding claims, wherein the average content of tamsulosin hydrochloride in the population of pellets is less than 0.15 to % w/w calculated on a dry pellet basis, preferably is from 0.10 to 0.145 % w/w calculated on a dry pellet basis.
Clause 44.-The pharmaceutical composition according to any one of the three preceding claims, wherein the tamsulosin in form of pellets exhibits a dissolution release profile in simulated gastric fluid using Ph. Eur. paddles method at 50 rpm which includes releasing less than 25 % of the tamsulosin during the first two hours.
Clause 45.-The pharmaceutical composition according to any one of the four preceding claims, wherein the tamsulosin in form of pellets exhibits a dissolution release profile in a phosphate buffer of pH 6.8 using Ph. Eur. paddles method at 100 rpm which includes releasing 15-45 % of the tamsulosin in 30 minutes.
Clause 46.-The pharmaceutical composition in the form of a hard shell capsule according to any one of the claims 41 or 42, wherein the average content of tamsulosin hydrochloride in the population of granules is less than 1.0 % calculated on a dry basis, preferably less than 0.4 to % w/w calculated on a dry basis, more preferably is from 0.15 to 0.35 % w/w calculated on a dry basis.
Clause 47.-The pharmaceutical composition according to any one of the claims 41, 42 or 46, wherein the tamsulosin in form of granules exhibits a dissolution release profile in a phosphate buffer of pH 6.8 using Ph. Eur. basket method at 50 rpm which includes releasing 15-30 % of the tamsulosin in 60 minutes.
Clause 48.-The pharmaceutical composition according to any one of the seven preceding claims, wherein the total volume of the hard shell capsule is equal or less than 1 ml, preferably from 0.6 ml to 0.95 ml, more preferably from 0.7 to 0.9 ml.
Clause 49.-The pharmaceutical composition according to any one of eight preceding claims, wherein the total volume of the soft gelatin capsule is equal or less than 0.4 ml, preferably from 0.2 to 0.37 ml, more preferably from 0.25 to 0.31 ml.
Clause 50.-A pharmaceutical batch of at least 100,000 soft gelatin capsules of the pharmaceutical composition according to any one of the preceding claims 32 to 40.
Clause 51.-The pharmaceutical batch according to the preceding claim, wherein the amount of 17-beta-N-[2,5-bis(trifluoromethyl)]phenylcarbamoyl-4-aza-5-alpha-androst-1-en-3-one in each soft gelatin capsule is from 0.4 to 0.6 mg.
Clause 52.-A blister comprising at least a unit dosage form of a pharmaceutical composition in the form of soft gelatin capsule according to any one of the preceding claims 32 to 40.
Clause 53.- The blister according to the preceding claim, wherein the blister is an aluminium/PVC/PVDC blister.
Clause 54.-A pharmaceutical composition in the form of a hard shell capsule comprising:
   (a) an immediate release soft gelatin capsule comprising the active agent 17-beta-N-[2,5-bis(trifluoromethyl)]phenylcarbamoyl-4-aza-5-alpha-androst-1-en-3-one and
   (b) tamsulosin in the form of pellets, wherein the average content of tamsulosin hydrochloride in the population of pellets is less than 0.15 to % w/w calculated on a dry pellet basis.
Clause 55.- The pharmaceutical composition in the form of a hard shell capsule according to the preceding claim, wherein the average content of tamsulosin hydrochloride in the population of pellets is from 0.10 to 0.145 % w/w calculated on a dry pellet basis.
Clause 56.-A pharmaceutical composition in the form of a hard shell capsule comprising:
   (a) an immediate release soft gelatin capsule comprising the active agent 17-beta-N-[2,5-bis(trifluoromethyl)]phenylcarbamoyl-4-aza-5-alpha-androst-1-en-3-one and
   (b) tamsulosin in the form of granules, wherein the average content of tamsulosin hydrochloride in the population of granules is less than 1.0 % calculated on a dry basis, preferably less than 0.4 to % w/w calculated on a dry basis.
Clause 57.-The pharmaceutical composition in the form of a hard shell capsule according to the preceding claim, wherein the average content of tamsulosin hydrochloride in the population of pellets is from 0.15 to 0.35 % w/w calculated on a dry basis.
Clause 58.-The pharmaceutical composition in the form of a hard shell capsule according to any one of the four preceding claims wherein the amount of the active agent 17-beta-N-[2,5-bis(trifluoromethyl)]phenylcarbamoyl-4-aza-5-alpha-androst-1-en-3-one is from 0.4 to 0.6 mg, preferably is 0.5 mg and the amount of tamsulosin is from 0.3 to 0.5 mg, preferably is 0.4 mg.
Clause 59.-The pharmaceutical composition according to any one of the five preceding claims, wherein the weight ratio active agent:total weight of the fill of the soft gelatin capsule is less than 1:600.
Clause 60.-The pharmaceutical composition according to the preceding claim wherein the weight ratio active agent:total weight of the fill of the soft gelatin capsule is between 1:240 to 1:600, preferably between 1:300 to 1:560, more preferably between 1:380 to 1:520.
Clause 61.-The pharmaceutical composition according to anyone of the seven preceding claims in wherein the soft gelatin capsule comprises less than 300 mg of a mixture of monoacylglycerols.
Clause 62.-The pharmaceutical composition according to the preceding claim wherein the soft gelatin capsule comprises from 120 to 300 mg of a mixture of monoacylglycerols, preferably from 150 to 280 mg of a mixture of monoacylglycerols, more preferably from 190 to 260 mg of a mixture of monoacylglycerols.
Clause 63.-The pharmaceutical composition according to any one of the two preceding claims wherein the mixture of monoacylglycerols is glycerol monocaprylocaprate.
Clause 64.- The pharmaceutical composition according to any one of the ten preceding claims wherein the shell of the soft gelatin capsule comprises an acid gelatin, preferably an acid gelatin having less than 150 g Bloom of gel strength.
Clause 65.-The pharmaceutical composition according to any one of the eleven preceding claims, wherein the gelatin is an acid gelatin having from 75 to 145 g Bloom of gel strength, preferably from 85 to 135 g Bloom of gel strength, more preferably from 95 to 125 g Bloom of gel strength.
Clause 66.-A pharmaceutical batch of at least 100,000 hard shell capsules of the pharmaceutical composition according to any one of the claims 28 to 31,41 to 49 or 54 to 65.
Clause 67.-The pharmaceutical batch according to the preceding claim, wherein the amount of 17-beta-N-[2,5-bis(trifluoromethyl)]phenylcarbamoyl-4-aza-5-alpha-androst-1-en-3-one in each hard capsule is from 0.4 to 0.6 mg and the amount of tamsulosin is from 0.3 to 0.5 mg.
Clause 68.-A packaged pharmaceutical composition wherein said packaged pharmaceutical composition is a high density polyethylene (HDPE) bottle or a blister, preferably the blister is an aluminium/aluminium blister comprising said bottle or blister at least a unit dosage form of a pharmaceutical composition according to any one of the claims 28 to 31,41 to 49 or 54 to 65.
Clause 69.-The HDPE bottle according to the preceding claim, wherein the HPDE bottle has a polypropylene child-resistant closure with induction-seal liners.
Clause 70.-A cardboard box with patient information leaflet comprising at least an HPDE bottle or an aluminium/aluminium blister, wherein said bottle or blister comprises with at least 4 hard capsules as defined in any one of claims 28 to 31,41 to 49 or 54 to 65.
Clause 71.-A process for manufacturing of soft gelatin capsules comprising the active agent 17-beta-N-[2,5-bis(trifluoromethyl)]phenylcarbamoyl-4-aza-5-alpha-androst-1-en-3-one comprising at least the following steps:
   i) preparing a soft gelatin capsule fill solution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-one; wherein the active agent has a water content higher than 0.05 % w/w;
   ii) encapsulating the fill obtained in step (i) in a shell to form filled soft gelatin capsules and;
   iii) optionally drying the soft gelatin capsules;
   preferably, wherein the temperature of the fill of steps (i) and (ii) is independently less than 40 °C.
Clause 72.-The process according to the preceding claim, wherein the active agent 17-beta-N-[2,5-bis(trifluoromethyl)]phenylcarbamoyl-4-aza-5-alpha-androst-1-en-3-one has a water content from 0.06 to 4 % w/w, preferably from 0.1 to 3 % w/w.
Clause 73.- The process according to any one of the two preceding claims, wherein the temperature of the fill of steps (i) and (ii) ranges independently between 20 to 35 °C.
Clause 74.-The process according to any one of the three preceding claims, wherein the active agent 17-beta-N-[2,5-bis(trifluoromethyl)]phenylcarbamoyl-4-aza-5-alpha-androst-1-en-3-one has a water content from 0.1 to 0.8 % w/w and the temperature of the fill of steps (i) and (ii) ranges independently between 28 to 34 °C, preferably the temperature of the fill of steps (i) and (ii) ranges independently between 30 to 32 °C.
Clause 75.-The process according to any one of claims 71 to 73, wherein the active agent 17-beta-N-[2,5-bis(trifluoromethyl)]phenylcarbamoyl-4-aza-5-alpha-androst-1-en-3-one has a water content from 0.8 to 2.5 % w/w and the temperature of the fill of steps (i) and (ii) ranges independently between 23 to 30 °C, preferably the temperature of the fill of steps (i) and (ii) ranges independently between 25 to 28 °C.
Clause 76.-The process according to any one of the five preceding claims, wherein step (ii) comprises encapsulating the soft gelatin capsule fill obtained in step (i) using a rotary die and producing the soft gelatin capsule.
Clause 77.-The process according to any one of the six preceding claims for manufacturing the pharmaceutical composition in the form of soft gelatin capsules as defined in any one of claims 1 to 20, claims 28 to 49 or claims 54 to 65.
Clause 78.-The pharmaceutical composition obtained by the process as described in any one of the seven preceding claims.
Clause 79.-A process for manufacturing a pharmaceutical composition in the form of an immediate release soft gelatin capsule comprising the active agent 17-beta-N-[2,5-bis(trifluoromethyl)]phenylcarbamoyl-4-aza-5-alpha-androst-1-en-3-one, wherein the soft gelatin capsule comprises, preferably consists essentially of a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; wherein the shell of the soft gelatin capsule comprises an acid gelatin, preferably an acid gelatin having less than 150 g Bloom of gel strength; comprising at least the following steps:
   i) forming the gelatin ribbons;
   ii) encapsulating the fill in the gelatin ribbons of step (i) to form filled soft gelatin capsules and;
   iii) optionally drying the soft gelatin capsules;
   wherein the temperature of the gelatin ribbons of step (i) and (ii) ranges between 35 to 50 °C.
Clause 80.-The process according to the preceding claim, wherein the temperature of the gelatin ribbons of step (i) and (ii) ranges between 40 to 45 °C.
Clause 81.-The process according to any of the two preceding claims, wherein the temperature of the gelatin ribbons of step (i) and (ii) ranges between 41 to 44 °C.
Clause 82.-The process according to any of the three preceding claims, wherein the gelatin is an acid gelatin having from 75 to 145 g Bloom of gel strength.
Clause 83.-The process according to any of the four preceding claims, wherein the gelatin is an acid gelatin having from 85 to 135 g Bloom of gel strength.
Clause 84.-The process according to any of the five preceding claims, wherein the gelatin is an acid gelatin having from 95 to 125 g Bloom of gel strength.
Clause 85.-The process according to any of the preceding process claims for manufacturing the pharmaceutical composition in the form of soft gelatin capsules as defined in any one of claims 1 to 20, claims 28 to 49 or claims 54 to 65.
Clause 86.-The pharmaceutical composition obtained by the process as described in any one of the eight preceding claims.
Clause 87.-The pharmaceutical composition according to the preceding claim, wherein the hardness of the immediate release soft gelatin capsules is from 5.0 to 15.0 N, preferably from 7.0 and 13.0 N, more preferably from 9.0 N and 11.0 N.
Clause 88.-A pharmaceutical batch of at least 100,000 soft gelatin capsules of the pharmaceutical composition according to any one of claims 78, 86 or 87.
Clause 89.-The pharmaceutical batch according to the preceding claim, wherein the amount of 17-beta-N-[2,5-bis(trifluoromethyl)]phenylcarbamoyl-4-aza-5-alpha-androst-1-en-3-one in each soft gelatin capsule is from 0.4 to 0.6 mg.
Clause 90.-A blister comprising at least a unit dosage form of a pharmaceutical composition according to any one of claims 78, 86 or 87.
Clause 91.-The blister comprising a unit dosage of the pharmaceutical composition according to the preceding claim, wherein the blister is an aluminium/PVC/PVDC blister.
Clause 92.- A cardboard box with patient information leaflet comprising at least a blister with at least 4 soft gelatin capsules as defined in any one of claims 32 to 40, 78, 86 or 87.
Clause 93.- Use of the pharmaceutical composition of any of the pharmaceutical composition claims for the manufacture of a medicament for the treatment of the symptoms of benign prostatic hyperplasia.

### DESCRIPTION OF THE FIGURES

Figure 1 depicts the immediate release dissolution profiles of soft gelatin capsules of batch TER 802-P1 at t=0 (TER 802 P1 T0) compared with dissolution profile after 6 months at 40 °C/75 % RH (TER 802 P1 T6 - 40/75); % dissolved vs time (min).
Figure 2 depicts the immediate release dissolution profile of a soft gelatin capsules of batch TER 802-P2 at t=0 (TER 802 P2 T0) compared with dissolution profile after 6 months at 40 °C/75 % RH (TER 802 P2 T6 - 40/75); % dissolved vs time (min).
Figure 3 depicts the immediate release dissolution profile of a soft gelatin capsules of batch TER 802-P3 at t=0 (TER 802 P3 T0) compared with dissolution profile after 6 months at 40 °C/75 % RH (TER 802 P3 T6 - 40/75); % dissolved vs time (min).
Figure 4 depicts the immediate release dissolution profile of a soft gelatin capsules of example 2b and 2c at t=0; % dissolved vs time (min).

### EXAMPLES

The following examples illustrate various embodiments of the invention and are not intended to limit the invention in any way.

The following example corresponds to a fill to be used in the manufacture of soft gelatin capsules. The fill is encapsulated to form soft gelatin capsules with shell compositions having gelatin, plastifier agent or agents and water as described below.

### Example 1a: Preparation soft gelatin capsule of dutasteride (3 batches TER 802-P1; TER 802-P2; TER 802-P3)

Fill composition of three batches:

| **Ingredients** | **mg** |
|---|---|
| Dutasteride | 0.5 |
| Glycerol monocaprylocaprate | 349.5 |
| Butylhydroxytoluene | 0.035 |
| Total fill content (mg/capsule) | 350.035 |

Capsule shell:

| **Ingredients** | **mg** |
|---|---|
| Gelatin A Gran 18 mesh 100/120 | 136.0-138.0 |
| Glycerol | 77.0-79.0 |
| Purified water | 121.0-123.0 |
| Titanium Dioxide (E171) | 1.0-2.0 |
| Iron oxide Yellow (E172) | 0.12-0.14 |

### Example 1b: Preparation soft gelatin capsule of dutasteride

Fill composition of three batches:

| **Ingredients** | **mg** |
|---|---|
| Dutasteride | 0.5 |
| Glycerol monocaprylocaprate | 349.5 |
| Butylhydroxytoluene | 0.035 |
| Total fill content (mg/capsule) | 350.035 |

Capsule shell:

| **Ingredients** | **mg** |
|---|---|
| Gelatin A Gran 18 mesh 100/120 | 155.0-157.0 |
| Glycerol | 88.0-90.0 |
| Purified water | 136.0-141.0 |
| Titanium Dioxide (E171) | 1.5-1.8 |
| Iron oxide Yellow (E172) | 0.13-0.15 |

### Example 2a: Preparation soft gelatin capsule of dutasteride small

Fill composition of three batches:

| **Ingredients** | **mg** |
|---|---|
| Dutasteride | 0.5 |
| Glycerol monocaprylocaprate | 300.0 |
| Butylhydroxytoluene | 0.035 |
| Total fill content (mg/capsule) | 300.535 |

Capsule shell:

| **Ingredients** | **mg** |
|---|---|
| Gelatin A Gran 18 mesh 100/120 | 155.0-157.0 |
| Glycerol | 88.0-90.0 |
| Purified water | 136.0-141.0 |
| Titanium Dioxide (E171) | 1.5-1.8 |
| Iron oxide Yellow (E172) | 0.13-0.15 |

### Example 2b: Preparation soft gelatin capsule of dutasteride small

Fill composition of three batches:

| **Ingredients** | **mg** |
|---|---|
| Dutasteride | 0.5 |
| Glycerol monocaprylocaprate | 250.0 |
| Butylhydroxytoluene | 0.035 |
| Total fill content (mg/capsule) | 250.535 |

Capsule shell:

| **Ingredients** | **mg** |
|---|---|
| Gelatin A Gran 18 mesh 100/120 | 155.0-157.0 |
| Glycerol | 88.0-90.0 |
| Purified water | 136.0-141.0 |
| Titanium Dioxide (E171) | 1.5-1.8 |
| Iron oxide Yellow (E172) | 0.13-0.15 |

### Example 2c: Preparation soft gelatin capsule of dutasteride small

Fill composition of three batches:

| **Ingredients** | **mg** |
|---|---|
| Dutasteride | 0.5 |
| Glycerol monocaprylocaprate | 200.0 |
| Butylhydroxytoluene | 0.035 |
| Total fill content (mg/capsule) | 200.535 |

Capsule shell:

| **Ingredients** | **mg** |
|---|---|
| Gelatin A Gran 18 mesh 100/120 | 155.0-157.0 |
| Glycerol | 88.0-90.0 |
| Purified water | 136.0-141.0 |
| Titanium Dioxide (E171) | 1.5-1.8 |
| Iron oxide Yellow (E172) | 0.13-0.15 |

### Manufacturing process of the soft gelatin capsules (batch of 100,000 soft gelatin capsules) of examples 1 and 2:

### 1. Preparation of the capsule fill

- Glycerol monocaprylocaprate was added to stainless steel tank equipped with cooling jacket to keep it at a temperature of 32-34 °C. It was stirred until completely dissolved.
- Butylhydroxytoluene (BHT) was added to the tank and stirred until completely dissolved.
- Finally dutasteride was added to the tank and stirred until completely dissolved.
- The resulting solution is kept at a temperature of less than 35 °C, preferably a range of temperature between 20 to 34 °C until the encapsulation process.

### 2. Preparation of the capsule shell

- Purified water and glycerol were weighed and mixed in the reactor.
- Heated until the mixture reached 80 °C.
- Gelatin was added and stirred at slow speed.
- The colorants previously dissolved in water were added to the mixture.

### 3. Capsulation process

- Lamination of the cover and thickness adjustment of each of the bands.
- Capsule filling.
- Drying of capsules.

### 4. Packaging

Primary packaging: the soft gelatin capsules were packed in blister of the following characteristics:
- 20 micron aluminium lacquer sealable to PVC or PVDC.
- PVC / PVDC white opaque.

### Example 3: Stability data of 3 batches of example 1

All the stability tests were performed with the soft gelatin capsules packaged in blister as described above in example 1. Dissolution test of dutasteride (% dutasteride) was determined at 60 min using USP Apparatus: II (Paddles), speed: 50 r.p.m. medium: 0.1 N HCl, containing 2 % sodium lauryl sulfate and wavelength 210 nm.

| **Batch N° TER 802-P1** | **t=0** | **25 °C / 60% RH-6 month** | **30 °C / 65% RH - 6 month** | **40 °C / 75% RH-6 month** |
|---|---|---|---|---|
| **Appearance** | opaque and yellow soft gelatin capsule containing an oily and yellowish liquid | opaque and yellow soft gelatin capsule containing an oily and yellowish liquid | opaque and yellow soft gelatin capsule containing an oily and yellowish liquid | opaque and yellow soft gelatin capsule containing an oily and yellowish liquid |
| **Dissolution (% dutasteride)** | 100.7 | 96.4 | 96.2 | 96.4 |
| **Assay (%)** | 99.4 | 98.9 | 100.7 | 100.7 |
| **RELATED SUBSTANCES** | | | | |
| Unknown individual impurities (%) | ND | ND | ND | ND |
| **Total impurities (%)** | ND | ND | ND | ND |
| | | | | |
| **Batch N° TER 802-P2** | **t=0** | **25 °C / 60% RH-6 month** | **30 °C / 65% RH - 6 month** | **40 °C / 75% RH-6 month** |
| **Appearance** | opaque and yellow soft gelatin capsule containing an oily and yellowish liquid | opaque and yellow soft gelatin capsule containing an oily and yellowish liquid | opaque and yellow soft gelatin capsule containing an oily and yellowish liquid | opaque and yellow soft gelatin capsule containing an oily and yellowish liquid |
| **Dissolution (% dutasteride)** | 101.7 | 99.5 | 110.1 | 99.4 |
| **Assay (%)** | 99.6 | 100.4 | 100.7 | 104.3 |
| **RELATED SUBSTANCES** | | | | |
| Unknown individual impurities (%) | ND | ND | ND | ND |
| **Total impurities (%)** | ND | ND | ND | ND |
| | | | | |
| **Batch N° TER 802-P3** | **t=0** | **25 °C / 60% RH-6 month** | **30 °C / 65% RH - 6 month** | **40 °C / 75% RH-6 month** |
| **Appearance** | Oblong, opaque and yellow soft gelatin capsule containing an oily and yellowish liquid | Oblong, opaque and yellow soft gelatin capsule containing an oily and yellowish liquid | Oblong, opaque and yellow soft gelatin capsule containing an oily and yellowish liquid | Oblong, opaque and yellow soft gelatin capsule containing an oily and yellowish liquid |
| **Dissolution (% dutasteride)** | 99.3 | 101.3 | 99.7 | 99.9 |
| **Assay (%)** | 99.9 | 99.3 | 101.9 | 104.7 |
| **RELATED SUBSTANCES** | | | | |
| Unknown individual impurities (%) | ND | ND | ND | ND |
| **Total impurities (%)** | ND | ND | ND | ND |

| | | | | |
|---|---|---|---|---|
| ND = not detected | | | | |

### Example 4: Dissolution profiles of 3 batches of example 1

Dissolution profile were performed from the 3 batches following the method USP Apparatus: II (Paddles), speed: 50 r.p.m. medium: 0.1N HCl, containing 2 % sodium lauryl sulfate and wavelength 210 nm during 60 minutes (sample at 10, 15, 30, 45 and 60 minutes) for soft gelatin capsules at t=0 and after 6 months of stability at 25 °C / 60 % RH; at 30 °C / 60 % RH; and at 40 °C / 75 % RH. The dissolution profile shows homogeneity between the different dissolution profiles and the dissolution profile of the soft gelatin capsules still corresponds to an immediate release composition.

Figure 1, 2 and 3 show the results of dissolution tests performed with soft gelatin capsules according to example 1 (3 batches: TER 802 P1, TER 802 P2, TER 802 P3) at t=0 compared with dissolution profile after 6 month at 40 °C/75 % RH.

**Table 1:**

| **Time (min)** | **% dissolved** | **% dissolved** | **% dissolved** | **% dissolved** |
|---|---|---|---|---|
| Batch | TER 802 P1 | TER 802 P1 T6 - 25/60 | TER 802 P1 T6-30/60 | TER 802 P1 T6 - 40/75 |
| Time | T0 | T=6 months | T=6 months | T=6 months |
| 0 | 0.0 | 0.0 | 0.0 | 0.0 |
| 10 | 45.6 | 54.9 | 55.8 | 35.4 |
| 15 | 84.5 | 85.0 | 80.2 | 76.7 |
| 30 | 98.0 | 96.6 | 95.5 | 94.0 |
| 45 | 99.1 | 96.5 | 96.2 | 97.3 |
| 60 | 100.5 | 96.4 | 96.2 | 96.4 |

**Table 2:**

| **Time (min)** | **% dissolved** | **% dissolved** | **% dissolved** | **% dissolved** |
|---|---|---|---|---|
| Batch | TER 802 P2 | TER 802 P2 T6 - 25 °C/60 | TER 802 P2 T6 - 30 °C/60 | TER 802 P2 T6 - 40 °C/75 |
| Time | T0 | T=6 months | T=6 months | T=6 months |
| 0 | 0.0 | 0.0 | 0.0 | 0.0 |
| 10 | 69.8 | 66.9 | 58.5 | 55.8 |
| 15 | 93.5 | 86.4 | 89.2 | 81.7 |
| 30 | 101.8 | 98.6 | 97.5 | 97.9 |
| 45 | 103.2 | 99.0 | 99.2 | 99.2 |
| 60 | 103.3 | 99.5 | 100.1 | 99.4 |

**Table 3:**

| **Time (min)** | **% dissolved** | **% dissolved** | **% dissolved** | **% dissolved** |
|---|---|---|---|---|
| Batch | TER 802 P3 | TER 802 P3 T6 - 25 °C/60 | TER 802 P3 T6 - 30 °C/60 | TER 802 P3 T6 - 40 °C/75 |
| Time | T0 | T=6 months | T=6 months | T=6 months |
| 0 | 0.0 | 0.0 | 0.0 | 0.0 |
| 10 | 46.6 | 38.8 | 49.8 | 23.9 |
| 15 | 82.6 | 80.6 | 78.7 | 75.7 |
| 30 | 98.8 | 99.2 | 97.3 | 96.2 |
| 45 | 99.8 | 101.3 | 98.5 | 98.4 |
| 60 | 100.3 | 101.3 | 99.7 | 99.9 |

### Example 5: Pharmaceutical composition of dutasteride and tamsulosin pellets

For purposes of the present invention, any capsule filling technology is believed to be adequate for preparing a fixed dose combination of the above described components. The fill order should not be considered critical to the present invention. Rather, the tamsulosin pellets and dutasteride soft gelatin capsules may be used to fill the capsule in either order.

The following examples illustrate the use of single pellets in small batches using pellets known from the state of art WO 2006055659, WO 2004056354 or WO 2010066268. Additionally, the use of soft gelatin capsules from Examples 2b and 2c are merely for illustration as any of the soft gelatin capsules produced in the examples can be used to create capsules with pellets that have different compositions.

**Table 4.**

| Component | Example 5a | Example 5b |
|---|---|---|
| Tamsulosin·HCl pellets | 285.3 mg/capsule | 285.3 mg/capsule |
| Dutasteride soft gelatin capsules Example 2b | 32,000 capsules | N/A |
| Dutasteride soft gelatin capsules Example 2c | N/A | 40,000 capsules |
| Hydroxypropyl methylcellulose capsule shell, size double 00 | 32,000 capsules | 40,000 capsules |

### Example 6: Stability data of pharmaceutical compositions of example 2b and 2c

All the stability tests were performed with the soft gelatin capsules packaged in blisters as described above in example 1. The dissolution test of dutasteride (% dutasteride) was determined at 60 min using USP Apparatus: II (Paddles), speed: 75 r.p.m. medium: 1 % w/v cetyltrimethylammonium bromide (CTAB) in 0.1 N HCl and wavelength: 210 nm.

| | **Ex 2b t=0** | **Ex 2c t=0** |
|---|---|---|
| **Appearance** | oblong soft gelatin capsule, opaque, yellow, containing an oily and yellowish liquid | oblong soft gelatin capsule, opaque, yellow, containing an oily and yellowish liquid |
| **Dissolution (% dutasteride)** | 96.2 | 96.8 |
| **Assay (%)** | Complies | Complies |
| **RELATED SUBSTANCES** | | |
| Unknown individual impurities (%) | 0.16 | 0.17 |
| **Total impurities (%)** | 0.16 | 0.17 |

### Example 7: Dissolution profiles of pharmaceutical compositions of example 2b and 2c

The dissolution test was determined using the method described in example 6 during 60 minutes (sample at 10, 15, 30, 45 and 60 minutes) for soft gelatin capsules at t=0.

The dissolution profile shows homogeneity between the different dissolution profiles and the dissolution profile of the soft gelatin capsules still corresponds to an immediate release composition.

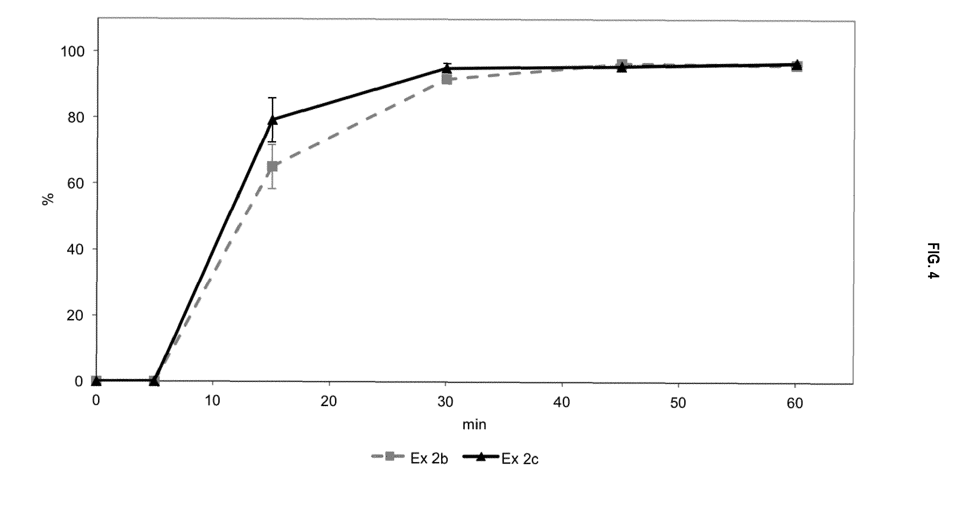

Figure 4 shows the results of the dissolution tests performed with soft gelatin capsules according to example 2b and 2c at t=0.

**Table 4:**

| **Time (min)** | **% dissolved** | **% dissolved** |
|---|---|---|
| Batch | Ex 2b | Ex 2c |
| Time | t=0 | t=0 |
| 0 | 0.0 | 0.0 |
| 15 | 65.1 | 79.3 |
| 30 | 91.9 | 95.2 |
| 45 | 96.5 | 95.8 |
| 60 | 96.2 | 96.8 |

### Example 8: Preparation soft gelatin capsule of dutasteride of the second aspect

Fill composition of three batches:

| **Ingredients** | **mg** |
|---|---|
| Dutasteride Anhydrous | 0.5 |
| Glycerol monocaprylocaprate | 250.0 |
| Butylhydroxytoluene | 0.035 |
| Total fill content (mg/capsule) | 250.535 |

Capsule shell:

| **Ingredients** | **mg** |
|---|---|
| Gelatin A Gran 18 mesh 100/120 | 155.0-157.0 |
| Glycerol | 88.0-90.0 |
| Purified water | 136.0-141.0 |
| Titanium Dioxide (E171) | 1.5-1.8 |
| Iron oxide Yellow (E172) | 0.13-0.15 |

### Manufacturing process of the soft gelatin capsules (batch of 100,000 soft gelatin capsules) of example 8:

### Preparation of the capsule fill

- Glycerol monocaprylocaprate was added to a stainless steel tank equipped with cooling jacket to keep it at a temperature of 20-23 °C. It was stirred until completely dissolved.
- Butylhydroxytoluene (BHT) was added to the tank and stirred until completely dissolved.
- Finally dutasteride anhydrous was added to the tank.
- The resulting solution was kept at a range of temperature of 20-23 °C. The solution was stirred, however, it was not possible to obtain a completely dissolved solution in this example. Water content of the active agent and the range of temperatures were studied to avoid dissolution problems.

### Example 9: Pharmaceutical composition of dutasteride and tamsulosin granules

For purposes of the present invention, any capsule filling technology is believed to be adequate for preparing a fixed dose combination of the above described components. The fill order should not be considered critical to the present invention. Rather, the tamsulosin granules and dutasteride soft gelatin capsules may be used to fill the capsule in either order.

The following examples illustrate the use of granules in small batches using granules known from the state of art WO 2006005512 (see, specifically Example 12 - table 3 of said PCT application). Additionally, the use of soft gelatin capsules from Examples 2b and 2c are merely for illustration as any of the soft gelatin capsules produced in the examples can be used to create capsules with granules that have different compositions.

Tamsulosin granules (WO2006005512, Example 12- table 3)

| **Ingredients** | **% w/w** |
|---|---|
| Tamsulosin HCl | 0.31 |
| Sodium alginate | 7.00 |
| Methacrylic acid/ethyl acrylate 1:1 copolymer | 58.94 |
| Glycerol dibehenate | 15.00 |
| Maltodextrin | 15.00 |
| Sodium lauryl sulfate | 1.15 |
| Macrogol 6000 | 1.69 |
| Polysorbate 80 | 0.65 |
| Sodium hydroxide | 0.05 |
| Simethicone | 0.01 |
| Colloidal anhydrous silica | 0.20 |

**Table 4.**

| Component | Example 5a | Example 5b |
|---|---|---|
| Tamsulosin·HCl granules | 130 mg/capsule | 130 mg/capsule |
| Dutasteride soft gelatin capsules Example 2b | 32,000 capsules | N/A |
| Dutasteride soft gelatin capsules Example 2c | N/A | 40,000 capsules |
| Hydroxypropyl methylcellulose capsule shell, size 0 elongated | 32,000 capsules | 40,000 capsules |

## Claims

1. A pharmaceutical composition in the form of a hard shell capsule comprising an immediate release soft gelatin capsule comprising the active agent 17-beta-N-[2,5-bis(trifluoromethyl)]phenylcarbamoyl-4-aza-5-alpha-androst-1-en-3-one and tamsulosin in the form of granules or powder, preferably in the form of granules.

2. The pharmaceutical composition according to the preceding claim wherein tamsulosin is in the form of granules and the average content of tamsulosin hydrochloride in the population of granules is less than 1.0 % calculated on a dry basis, preferably less than 0.4 % w/w calculated on a dry basis, more preferably is from 0.15 to 0.35 % w/w calculated on a dry basis.

3. The pharmaceutical composition according to any one of the preceding claims wherein the soft gelatin capsule comprises, preferably consists essentially of, a fill and a capsule shell: wherein the fill of the soft gelatin capsule comprises the active agent 17-beta-N-[2,5-bis(trifluoromethyl)]phenylcarbamoyl-4-aza-5-alpha-androst-1-en-3-one, and at least a pharmaceutically acceptable excipient or excipients; and wherein the shell of the soft gelatin capsule comprises an acid gelatin, preferably an acid gelatin having less than 150 g Bloom of gel strength, more preferably from 85 to 135 g Bloom of gel strength, even more preferably from 95 to 125 g Bloom of gel strength; and wherein the pharmaceutical composition is suitable for being stored up to 40 °C.

4. The pharmaceutical composition according to any one of the preceding claims, wherein the weight ratio active agent:total weight of the fill of the soft gelatin capsule is less than 1:600, preferably between 1:300 to 1:560, more preferably between 1:380 to 1:520.

5. The pharmaceutical composition according to any one of the preceding claims, wherein the fill of the soft gelatin capsule further comprises at least a pharmaceutically acceptable excipient, such as glycerol monocaprylocaprate and butylhydroxytoluene, preferably the fill of the soft gelatin capsule further comprises glycerol monocaprylocaprate and at least an antioxidant, more preferably butylhydroxytoluene; and/or wherein the soft gelatin capsule comprises less than 300 mg of a mixture of monoacylglycerols, preferably from 120 to 300 mg of a mixture of monoacylglycerols, more preferably from 190 to 260 mg of a mixture of monoacylglycerols; even more preferably the mixture of monoacylglycerols is glycerol monocaprylocaprate.

6. The pharmaceutical composition according to any one of the preceding claims, wherein the tamsulosin in form of granules exhibits a dissolution release profile in a phosphate buffer of pH 6.8 using Ph. Eur. basket method at 50 rpm which includes releasing 15-30 % of the tamsulosin in 60 minutes.

7. The pharmaceutical composition according to any one of the preceding claims, wherein the total volume of the hard shell capsule is equal or less than 1 ml, preferably from 0.6 ml to 0.95 ml, more preferably from 0.7 to 0.9 ml; and/or wherein the total volume of the soft gelatin capsule is equal or less than 0.4 ml, preferably from 0.2 to 0.37 ml, more preferably from 0.25 to 0.31 ml.

8. The pharmaceutical composition according to any one of the the preceding claims, wherein the amount of the active agent 17-beta-N-[2,5-bis(trifluoromethyl)]phenylcarbamoyl-4-aza-5-alpha-androst-1-en-3-one is from 0.4 to 0.6 mg, preferably is 0.5 mg; and the amount of tamsulosin is from 0.3 to 0.5 mg, preferably is 0.4 mg.

9. A process for manufacturing the pharmaceutical composition as defined in any one of the preceding claims, comprising at least the following steps:
i) preparing a soft gelatin capsule fill solution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-one; wherein the active agent has a water content higher than 0.05 % w/w, preferably from 0.06 to 4 % w/w, more preferably from 0.1 to 3 % w/w;
ii) encapsulating the fill obtained in step (i) in a shell to form filled soft gelatin capsules;
iii) optionally, drying the soft gelatin capsules obtained in step (ii);
preferably, wherein the temperature of the fill of steps (i) and (ii) is independently less than 40 °C, more preferably the temperature of the fill of steps (i) and (ii) ranges independently between 20 to 35 °C; and
iv) filling the hard shell capsule with tamsulosin granules and the soft gelatin capsules obtained in step (ii) or (iii).

10. The process according to the preceding claim, wherein the active agent 17-beta-N-[2,5-bis(trifluoromethyl)]phenylcarbamoyl-4-aza-5-alpha-androst-1-en-3-one has a water content from 0.1 to 0.8 % w/w and the temperature of the fill of steps (i) and (ii) ranges independently between 28 to 34 °C, preferably the temperature of the fill of steps (i) and (ii) ranges independently between 30 to 32 °C; and/or wherein the active agent 17-beta-N-[2,5-bis(trifluoromethyl)]phenylcarbamoyl-4-aza-5-alpha-androst-1-en-3-one has a water content from 0.8 to 2.5 % w/w and the temperature of the fill of steps (i) and (ii) ranges independently between 23 to 30 °C, preferably the temperature of the fill of steps (i) and (ii) ranges independently between 25 to 28 °C.

11. A process for manufacturing a the pharmaceutical composition as defined in any one of claims 1 to 8, comprising at least the following steps:
i) forming gelatin ribbons;
ii) encapsulating the fill solution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-onesolution comprising glycerol monocaprylocaprate; at least an antioxidant, preferably butylhydroxytoluene; and the active agent 17-beta-N-[2,5-bis(trifluoromethyl)]phenylcarbamoyl-4-aza-5-alpha-androst-1-en-3-one in the gelatin ribbons of step (i) to form filled soft gelatin capsules;
iii) optionally, drying the soft gelatin capsules obtained in step (ii);
wherein the temperature of the gelatin ribbons of step (i) and (ii) ranges between 35 to 50 °C, preferably ranges between 40 to 45 °C; and
iv) filling the hard shell capsule with tamsulosin granules and the soft gelatin capsules obtained in step (ii) or (iii).

12. The pharmaceutical composition obtained by the process as described in any one of the three preceding claims.

13. A pharmaceutical batch of at least 100,000 hard shell capsules of the pharmaceutical composition according to any one of claims 1 to 8 or 12, preferably wherein the amount of 17-beta-N-[2,5-bis(trifluoromethyl)]phenylcarbamoyl-4-aza-5-alpha-androst-1-en-3-one in each hard capsule is from 0.4 to 0.6 mg and the amount of tamsulosin is from 0.3 to 0.5 mg.

14. A packaged pharmaceutical composition wherein said packaged pharmaceutical composition is a high density polyethylene (HDPE) bottle or a blister, preferably the blister is an aluminium/aluminium blister comprising said bottle or blister at least a unit dosage form of a pharmaceutical composition according to any one of claims 1 to 8 or 12, preferably, when it is packaged in a HPDE bottle, the HPDE bottle has a polypropylene child-resistant closure with induction-seal liners; or a cardboard box with patient information leaflet comprising at least an HPDE bottle or an aluminium/aluminium blister, wherein said bottle or blister comprises at least 4 hard capsules as defined in any one of claims 1 to 8 or 12.

15. Use of the pharmaceutical composition according to any one of claims 1 to 8 or 12, or the pharmaceutical batch according to claim 13, for the treatment of the symptoms of benign prostatic hyperplasia.
